(19) Europäisches Patentamt
European Patent Office
Office européen des brevets

(11) **EP 1 423 138 B1**

(12) **EUROPEAN PATENT SPECIFICATION**

(45) Date of publication and mention
of the grant of the patent:
**24.12.2014 Bulletin 2014/52**

(21) Application number: **02767299.7**

(22) Date of filing: **01.08.2002**

(51) Int Cl.:
*A61K 38/17* (2006.01)      *A61K 38/21* (2006.01)
*A61K 39/395* (2006.01)      *A61K 48/00* (2006.01)
*A61K 47/48* (2006.01)      *A61P 37/00* (2006.01)

(86) International application number:
**PCT/EP2002/008591**

(87) International publication number:
**WO 2003/013577 (20.02.2003 Gazette 2003/08)**

(54) **USE OF IL-18 INHIBITORS IN HYPERSENSITIVITY DISORDERS**

VERWENDUNG VON IL-18 INHIBITOREN BEI ÜBEREMPFINDLICHKEITSSTÖRUNGEN

UTILISATION D'INHIBITEURS DE IL-18 POUR LE TRAITEMENT DE TROUBLES
D'HYPERSENSIBILITE

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(30) Priority: **10.08.2001  EP 01118811
20.06.2002  EP 02100735**

(43) Date of publication of application:
**02.06.2004  Bulletin 2004/23**

(73) Proprietor: **Merck Serono SA
1267 Coinsins, Vaud (CH)**

(72) Inventors:
• **CHVATCHKO, Yolande
CH-1232 Confignon (CH)**
• **KOSCO-VILBOIS, Marie
F-74270 Minzier (FR)**

(74) Representative: **Weiss, Wolfgang et al
Weickmann & Weickmann
Patentanwälte
Postfach 86 08 20
81635 München (DE)**

(56) References cited:
**EP-A- 0 864 585      EP-A- 0 974 600
EP-A- 1 110 969      WO-A-00/71719
WO-A-99/46248**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention is in the field of allergies. More specifically, it relates to the use of an inhibitor of IL-18 as defined in claim 1 for the treatment and/or prevention of a type IV hypersensitivity disorder and in particular of disorders involving delayed type hypersensitivity reactions of the human body.

BACKGROUND OF THE INVENTION

The term allergy or hypersensitivity is applied when an adaptive immune response occurs in an inappropriate form. Allergic or hypersensitivity reactions are the result of normally beneficial immune responses acting inappropriately to foreign antigens (usually environmental macromolecules) and sometimes cause inflammatory reactions and tissue damage. In these situations, a normally harmless environmental stimulus, called an allergen, triggers an immune response, which upon re-exposure, is re-activated to generate pathological damage.

Hypersensitivity reactions are damaging immunological reactions to extrinsic antigens. Many classifications of hypersensitivity exist. Some are based on the time required for symptoms or skin test reactions to appear after exposure to an antigen (e.g., immediate and delayed hypersensitivity), on the type of antigen (e.g., drug reactions), or on the nature of organ involvement. Classifications are generally oversimplified and do not take into account that more than one type of immune response may be occurring or that more than one type may be necessary to produce immunological injury. The most widely used classification is the following:

Type I or immediate hypersensitivity is IgE-mediated. It is also called common allergy. Immediate hypersensitivity (type I) reactions are due to the binding between antigen and IgE on mast cells or basophils.

Disorders with type I hypersensitivity reactions are also called atopic diseases, they include allergic rhinitis, allergic conjunctivitis, atopic dermatitis, allergic extrinsic asthma, urticaria, systemic anaphylaxis, for example. The incidence of asthma has increased markedly, although the causes are largely unknown. Recently, a marked increase in type I reactions has been noted in relation to exposure to water-soluble proteins in latex products (e.g., rubber gloves, dental dams, condoms, tubing for respiratory equipment, catheters, particularly among medical personnel and patients exposed to latex and children with spina bifida and urogenital birth defects. Common reactions to latex are urticaria, angioedema, conjunctivitis, rhinitis, bronchospasm, and anaphylaxis.

Patients with atopic diseases (including atopic dermatitis) usually have an inherited predisposition for developing IgE antibody-mediated hypersensitivity to inhaled and ingested substances (allergens) that are harmless to people who are not atopic. Except in atopic dermatitis, IgE antibodies usually mediate hypersensitivity.

Type II or cytotoxic hypersensitivity involves cytolytic actions mediated by antibody, complement, and/or cellular mechanisms. The target in type II reactions is a cell surface, and cellular damage or death is the result. Antibody to cell-bound antigen (type II) causes cell destruction by activating complement or promoting phagocytosis. Examples of cell injury in which antibody reacts with antigenic components of a cell are Coombs'-positive hemolytic anemias, antibody-induced thrombocytopenic purpura, leukopenia, pemphigus, pemphigoid, Goodpasture's syndrome, and pernicious anemia. These reactions occur in patients receiving incompatible transfusions, in hemolytic disease of the newborn, and in neonatal thrombocytopenia, and they also may play a part in multisystem hypersensitivity diseases (e.g. systemic lupus erythematosus, SLE).

The mechanism of injury is best exemplified by the effect on red blood cells. In hemolytic anemias, the red blood cells are destroyed either by intravascular hemolysis or by macrophage phagocytosis, predominantly within the spleen. In vitro studies have shown that in the presence of complement some complement-binding antibodies (e.g. the blood group antibodies anti-A and anti-B) cause rapid hemolysis. Others (e.g. anti-LE antibodies) cause a slow cell lysis; still others do not damage cells directly but cause their adherence to and destruction by phagocytes. In contrast, Rh antibodies on red blood cells do not activate complement, and they destroy cells predominantly by extravascular phagocytosis. Examples in which the antigen is a component of tissue are early acute (hyperacute) graft rejection of a transplanted kidney, which is due to the presence of antibody to vascular endothelium, and Goodpasture's syndrome, which is due to reaction of antibody with glomerular and alveolar basement membrane endothelium. In experimental Goodpasture's syndrome, complement is an important mediator of injury, but the role of complement has not been clearly determined in early acute graft rejection.

Examples of reactions due to haptenic coupling with cells or tissue include many of the drug hypersensitivity reactions (e.g. penicillin-induced hemolytic anemia, see below).

Anti-receptor hypersensitivity reactions alter cellular function as a result of the binding of antibody to membrane receptors. In many diseases (e.g., myasthenia gravis, Graves' disease, insulin-resistant diabetes), antibodies to cell membrane receptors have been reported. In some diabetic patients with extreme insulin resistance, antibodies to insulin receptors have been shown, thus preventing the binding of insulin to its receptor. In patients with Graves' disease, an antibody to the thyroid-stimulating hormone (TSH) receptor has been identified that simulates the effect of TSH on its receptor, resulting in hyperthyroidism.

Type III mechanisms involve mostly antibodies forming immune complexes with antigen. Circulating complexes activate complement, attach to red blood cells (which are then phagocytosed in the spleen), leave the circulation and trigger inflammation in tissue spaces (Arthus reaction), or are phagocytosed by macrophages which present antigen, release cytokines and activate B and T-cells. IgE, IgA, IgG, and IgM all form complexes with antigen. Type III reactions result from deposition of immune complexes in tissues, particularly the skin, joints and kidneys. Chronic immune complex nephritis accounts for most cases of glomerulonephritis in humans. Conditions in which immune complexes (ICs) appear to play some role are serum sickness due to serum, drugs, or viral hepatitis antigen; systemic lupus erythematosus; rheumatoid arthritis; polyarteritis; cryoglobulinemia; hypersensitivity pneumonitis; bronchopulmonary aspergillosis; acute glomerulonephritis; chronic membranoproliferative glomerulonephritis; and associated renal disease. In bronchopulmonary aspergillosis, drug- or serum-induced serum sickness, and some forms of renal disease, an IgE-mediated reaction is thought to precede the type III reaction.

The standard animal models of type III reactions are the local Arthus reaction and experimental serum sickness. In the Arthus reaction (typically a local skin reaction), animals are first hyperimmunized to induce large amounts of circulating IgG antibodies and then are given a small amount of antigen intradermally. The antigen precipitates with the excess IgG and activates complement, so that a highly inflammatory, edematous, painful local lesion rapidly appears (by 4 to 6 h) and may progress to a sterile abscess containing many polymorphonuclear cells, and then to tissue necrosis. A necrotizing vasculitis with occluded arteriolar lumina can be seen microscopically. No lag time precedes the reaction because antibody is present already.

Type I, II and III reactions are caused by antibodies. Type IV reactions are caused by T-lymphocytes.

Type IV hypersensitivity, involving cell-mediated reactions, generally take 12 or more hours to develop and are based on activated immune cell networks. Inflammation is the basic tissue pattern and chronic inflammatory disease may be the result. Type IV hypersensitivity is also called delayed-type hypersensitivity (type IV) or DTH. Reactions are mediated by interleukin-2, interferon-$\gamma$ and other cytokines released by T-lymphocytes. In DTH, T-lymphocytes react with antigen and release interleukin-9, interferon-$\gamma$ and other cytokines. Once T-cells have been sensitised by primary exposure, secondary challenge is followed by a delayed-type hypersensitivity reaction, a local inflammatory response which takes 2-3 days to develop clinically. Histologically, these reactions consist of infiltrating T-lymphocytes, macrophages and occasional eosinophils. Experimentally, DTH can be transferred by T-lymphocytes but not by serum, i.e. antibodies are not involved.

DTH may result from the normal cell-mediated immune response to infection with viruses, fungi and certain bacterial, notably Mycobacterium tuberculosis and Mycobacterium leprae. If macrophages are unable to destroy ingested organisms, they may undergo differentiation into epitheloid cells or multinucleate giant cells. A collection of these cells forms a granuloma. Local tissue damage is an unwanted side-effect of this otherwise protective immune response. If the DTH response is absent or impaired, however, T-lymphocytes are unable to localise the invading micro-organism and patients develop invasive aggressive disseminated disease, such as acute tuberculosis.

Contact dermatitis to occupational and other antigens is also a type IV reaction. Agents, which cause this are usually of comparatively low molecular weight (< 1 kD) and not immunogenic on their own, instead, they are highly reactive molecules that bind covalently to skin or tissue proteins. The sensitising chemical is known as a hapten and the host protein it combines with as the carrier. The range of potential sensitising antigens is wide. Two phases of pathogenesis are recognised: the induction phase and the elicitation phase. In the induction phase, antigen-presenting cells in the skin, known as Langerhans' cells, bind the hapten-carrier protein complex and present it to T-lymphocytes in association with MHC class II antigen. Induction of T-cells may occur after months of exposure to small amounts of antigen. Re-exposure to the relevant antigen triggers the elicitation phase where effector cells migrate to the skin to meet the protein complex presented by Langerhans' cells in the epidermis with consequent cytokine release and skin inflammation. The diagnosis of the offending agent is made by patch testing. A suspected contact sensitizer is applied on the patient's back and covered for 48 hours. The reaction site is inspected after 2 and 24 hours. In a positive response there is inflammation and induration at the test site. '

Delayed type hypersensitivity is also a key mechanism determining the rejection of transplanted test organs.

Some clinical conditions in which type IV reactions are believed to be important are contact dermatitis, hypersensitivity pneumonitis, allograft rejection, granulomas due to intracellular organisms, some forms of drug sensitivity, thyroiditis, and encephalomyelitis after rabies vaccination. Evidence for the last two is based on experimental models, and in human disease on the appearance of lymphocytes in the inflammatory exudate of the thyroid and brain.

Dermatitis is also called eczema. It relates to a superficial skin inflammation, characterized histologically by epidermal edema and clinically by vesicles (when acute), poorly marginated redness, edema, oozing, crusting, scaling, usually pruritus, and lichenification caused by scratching or rubbing.

Often, eczema refers to vesicular dermatitis, but sometimes the term is restricted eczema to mean chronic dermatitis. Some also refer to dermatitis as spongiotic dermatitis because spongiosis (intraepidermal edema) is a histologic feature

Contact Dermatitis is an acute or chronic inflammation, often asymmetric or oddly shaped, produced by substances contacting the skin and causing toxic (irritant) or allergic reactions.

Diagnosis of hypersensitivitiy reactions depend on the type of reaction involved.

A type IV reaction can be suspected when an inflammatory reaction is characterised histologically by perivascular lymphocytes and macrophages. Delayed hypersensitivity skin tests and patch tests are the most readily available methods of testing for delayed hypersensitivity.

To prevent exacerbation of contact dermatitis, patch tests are performed after the contact dermatitis has cleared. The suspected allergen (in appropriate concentration) is applied to the skin under a non-absorbent adhesive patch and left for 48 h. If burning or itching develops earlier, the patch is removed. A positive test consists of erythema with some induration and, occasionally, vesicle formation. Because some reactions do not appear until after the patches are removed, the sites are re-inspected at 72 and 96 h.

Hypersensitivity may also occur as a reaction to drugs. Before attributing a given reaction to a drug, it should be noted that placebos also may cause a wide variety of symptoms and even objective signs, such as skin rashes. Nevertheless, true drug reactions constitute a major medical problem.

In drug intolerance, the adverse reaction develops on the first use of the drug. It may be the same toxic reaction ordinarily expected at higher doses, or it may be an exaggeration of a common mild side effect (e.g. antihistaminic sedation). Idiosyncrasy is a condition in which the adverse reaction on first use of the drug is pharmacologically unexpected and unique.

Characteristics of allergic reactions to drugs include IgE-mediated reactions occurring only after the patient has been exposed to the drug (not necessarily for therapy) one or more times without incident. Once hypersensitivity has developed, the reaction can be produced by doses far below therapeutic amounts, and usually below those levels that produce idiosyncratic reactions. Clinical features are restricted in their manifestations. Skin rashes (particularly urticaria), serum sickness-like syndrome, unexpected fever, anaphylaxis, and eosinophilic pulmonary infiltrates appearing during drug therapy are usually due to hypersensitivity; some cases of anemia, thrombocytopenia, or agranulocytosis. Rarely, vasculitis develops after repeated exposure to a drug (e.g. sulfonamides, iodides, penicillin), and interstitial nephritis (e.g. methicillin) and liver damage (e.g. halothane) have been reported in circumstances consistent with development of specific hypersensitivity.

The most serious example of drug hypersensitivity is anaphylaxis. However, the most common drug reaction, by far, is a morbilliform rash, again of unknown etiology. Fever and urticarial reactions are also relatively common consequences of drug allergy. When animal sera were used for therapy, serum sickness was a complication, but animal sera are rarely used today. A serious serum sickness-like syndrome of unknown pathogenesis without high levels of circulating IgG antibody but usually associated with IgE antibodies can occur, especially with drugs such as penicillin.

Drug hypersensitivity reactions are based on the capacity of proteins and large polypeptide drugs to stimulate specific antibody production by straightforward immunologic mechanisms. Perhaps the smallest molecule that is potentially antigenic is glucagon, with a molecular weight of about 3500. Most drug molecules are much smaller and cannot act alone as antigens. However, as haptens, some bind covalently to proteins, and the resulting conjugates stimulate antibody production specific for the drug. The drug, or one of its metabolites, is chemically reactive with protein. The serum-protein binding common to many drugs is much weaker and of insufficient strength for antigenicity.

The specific immunologic reaction has been determined only for benzylpenicillin. This drug does not bind firmly enough to tissue or serum proteins to form an antigenic complex, but its major degradation product, benzylpenicillenic acid, can combine with tissue proteins to form benzylpenicilloyl (BPO), the major antigenic determinant of penicillin. Several minor antigenic determinants are formed in relatively small amounts by mechanisms that are less well defined. Hypersensitivity reactions (I, II, III, IV) most commonly involve the BPO determinant. IgE antibodies to minor determinants may be responsible in some patients for anaphylaxis and urticaria. IgG antibodies have been found to the major but not to the minor determinants. They may act as "blocking antibodies" to BPO, modifying or even preventing a reaction to BPO, while the lack of blocking IgG antibodies to the minor determinants may explain the ability of these determinants to induce anaphylaxis.

All semi-synthetic penicillins (e.g. amoxicillin, carbenicillin, ticarcillin) potentially cross-react with penicillin, so that penicillin-sensitive patients often react to them as well. Cross-reactions occur with cephalosporins to a lesser degree. Treatment with a cephalosporin should be started with great caution if the patient has a history of a severe reaction (e.g. anaphylaxis) to penicillin.

Hematologic antibody-mediated (cytotoxic, type II) drug reactions may develop by any of three mechanisms: In penicillin-induced anemia, the antibody reacts with the hapten, which is firmly bound to the red blood cell membrane, producing agglutination and increased destruction of red blood cells. In stibophen- and quinidine-induced thrombocytopenia the drug forms a soluble complex with its specific antibody. The complex then reacts with nearby platelets (the "innocent bystander" target cells) and activates complement, which alone remains on the platelet membrane and induces cell lysis. In other hemolytic anemias, the drug (e.g. methyldopa) appears to alter the red blood cells surface chemically, thereby uncovering an antigen that induces and then reacts with an autoantibody, usually of Rh specificity.

Toxic-idiosyncratic and anaphylactic reactions are sufficiently unique in kind or in time such that the offending drug is usually easily identified. Serum sickness-type reactions are most often due to the penicillins, but occasionally sulfonamides, hydralazine, sulfonylureas, or thiazides are responsible. Photosensitization is characteristic of chlorpromazine, certain antiseptics in soaps, sulfonamides, psoralens, demeclocycline, and griseofulvin. All drugs except those deemed absolutely essential should be stopped. When drug fever is suspected, the most likely drug is stopped (e.g. allopurinol, penicillin, isoniazid, sulfonamides, barbiturates, quinidine). Reduction in fever within 48 h strongly suggests that drug. If fever is accompanied by granulocytopenia, drug toxicity is more likely than allergy and is much more serious.

Allergic pulmonary reactions to drugs are usually infiltrative, with eosinophilia, and can be produced by gold salts, penicillin, and sulfonamides, among others. The most common cause of an acute infiltrative pulmonary reaction is nitrofurantoin. This is probably allergic but usually not eosinophilic.

Hepatic reactions may be primarily cholestatic (phenothiazines and erythromycin estolate are most frequently involved) or hepatocellular (allopurinol, hydantoins, gold salts, isoniazid, sulfonamides, valproic acid, and many others). The usual allergic renal reaction is interstitial nephritis, most commonly due to methicillin; other antimicrobials and cimetidine have also been implicated.

A syndrome similar to systemic lupus erythematosus can be produced by several drugs, most commonly hydralazine and procainamide. The syndrome is associated with a positive test for antinuclear antibody and is relatively benign, sparing the kidneys and CNS. Penicillamine can produce SLE and other autoimmune diseases, most notably myasthenia gravis.

In 1989, an endotoxin-induced serum activity that induced interferon-$\gamma$ (IFN-$\gamma$) obtained from mouse spleen cells was described (Nakamura et al., 1989). This serum activity functioned not as a direct inducer of IFN-$\gamma$ but rather as a co-stimulant together with IL-2 or mitogens. An attempt to purify the activity from post-endotoxin mouse serum revealed an apparently homogeneous 50-55 kDa protein. Since other cytokines can act as co-stimulants for IFN-$\gamma$ production, the failure of neutralizing antibodies to IL-1, IL-4, IL-5, IL-6, or TNF to neutralize the serum activity suggested it was a distinct factor. In 1995, the same scientists demonstrated that the endotoxin-induced co-stimulant for IFN-$\gamma$ production was present in extracts of livers from mice preconditioned with P. acnes (Okamura et al., 1995). In this model, the hepatic macrophage population (Kupffer cells) expand and in these mice, a low dose of bacterial lipopolysaccharide (LPS), which in non-preconditioned mice is not lethal, becomes lethal. The factor, named IFN-$\gamma$ -inducing factor (IGIF) and later designated interleukin-18 (IL-18), was purified to homogeneity from 1,200 grams of P. acnes-treated mouse livers. Degenerate oligonucleotides derived from amino acid sequences of purified IL-18 were used to clone a murine IL-18 cDNA. IL-18 is an 18-19 kDa protein of 157 amino acids, which has no obvious similarities to any peptide in the databases. Messenger RNAs for IL-18 and interleukin-12 (IL-12) are readily detected in Kupffer cells and activated macrophages. Recombinant IL-18 induces IFN-gamma more potently than does IL-12, apparently through a separate pathway (Micallef et al., 1996). Similar to the endotoxin-induced serum activity, IL-18 does not induce IFN-$\gamma$ by itself, but functions primarily as a co-stimulant with mitogens or IL-2. IL-18 enhances T cell proliferation, apparently through an IL-2-dependent pathway, and enhances Th1 cytokine production in vitro and exhibits synergism when combined with IL-12 in terms of enhanced IFN-$\gamma$ production (Maliszewski et al., 1990).

After the murine form was cloned, the human cDNA sequence for IL-18 was reported in 1996 (Ushio et al., 1996).

By cloning IL-18 from affected tissues and studying IL-18 gene expression, a close association of this cytokine with an autoimmune disease was found. The non-obese diabetic (NOD) mouse spontaneously develops autoimmune insulitis and diabetes, which can be accelerated and synchronized by a single injection of cyclophosphamide. IL-18 mRNA was demonstrated by reverse transcriptase PCR in NOD mouse pancreas during early stages of insulitis. Levels of IL-18 mRNA increased rapidly after cyclophosphamide treatment, and preceded a rise in IFN-$\gamma$ mRNA, and subsequently diabetes. Interestingly, these kinetics mimic that of IL-12-p40 mRNA, resulting in a close correlation of individual mRNA levels. Cloning of the IL-18 cDNA from pancreas RNA followed by sequencing revealed identity with the IL-18 sequence cloned from Kupffer cells and in vivo pre-activated macrophages. Also NOD mouse macrophages responded to cyclophosphamide with IL-18 gene expression while macrophages from Balb/c mice treated in parallel did not. Therefore, IL-18 expression is abnormally regulated in autoimmune NOD mice and closely associated with diabetes development (Rothe et al., 1997).

IL-18 plays a potential role in immunoregulation or in inflammation by augmenting the functional activity of Fas ligand on Th1 cells (Conti et al., 1997). IL-18 is also expressed in the adrenal cortex and therefore might be a secreted neuro-immunomodulator, playing an important role in orchestrating the immune system following a stressful experience (Chater, 1986).

In vivo, IL-18 is formed by cleavage of pro-IL-18, and its endogenous activity appears to account for IFN-$\gamma$ production in P. acnes and LPS-mediated lethality. Mature IL-18 is produced from its precursor by the IL-1$\beta$ converting enzyme (IL-1beta-converting enzyme, ICE, caspase-1).

The IL-18 receptor consists of at least two components, co-operating in ligand binding. High- and low-affinity binding sites for IL-18 were found in murine IL-12 stimulated T cells (Yoshimoto et al., 1998), suggesting a multiple chain receptor complex. Two receptor subunits have been identified so far, both belonging to the IL-1 receptor family (Parnet et al., 1996). The signal transduction of IL-18 involves activation of NF-κB (DiDonato et al., 1997).

Recently, a soluble protein having a high affinity for IL-18 has been isolated from human urine, and the human and mouse cDNAs were described (Novick et al., 1999; WO 99/09063). The protein has been designated IL-18 binding protein (IL-18BP).

IL-18BP is not the extracellular domain of one of the known IL18 receptors, but a secreted, naturally circulating protein. It belongs to a novel family of secreted proteins. The family further includes several Poxvirus-encoded proteins which have a high homology to IL-18BP (Novick et al., 1999). IL-18BP is constitutively expressed in the spleen, belongs to the immunoglobulin superfamily, and has limited homology to the IL-1 type II receptor. Its gene was localized on human chromosome 11q13, and no exon coding for a transmembrane domain was found in an 8.3 kb genomic sequence (Novick et al., 1999).

Four human and two mouse isoforms of IL-18BP, resulting from mRNA splicing and found in various cDNA libraries and have been expressed, purified, and assessed for binding and neutralization of IL-18 biological activities (Kim et al., 2000). Human IL-18BP isoform a (IL-18BPa) exhibited the greatest affinity for IL-18 with a rapid on-rate, a slow off-rate, and a dissociation constant (K(d)) of 399 pM. IL-18BPc shares the Ig domain of IL-18BPa except for the 29 C-terminal amino acids; the K(d) of IL-18BPc is 10-fold less (2.94 nM). Nevertheless, IL-18BPa and IL-18BPc neutralize IL-18 >95% at a molar excess of two. IL-18BPb and IL-18BPd isoforms lack a complete Ig domain and lack the ability to bind or neutralize IL-18. Murine IL-18BPc and IL-18BPd isoforms, possessing the identical Ig domain, also neutralize >95% murine IL-18 at a molar excess of two. However, murine IL-18BPd, which shares a common C-terminal motif with human IL-18BPa, also neutralizes human IL-18. Molecular modeling identified a large mixed electrostatic and hydrophobic binding site in the Ig domain of IL-18BP, which could account for its high affinity binding to the ligand (Kim et al., 2000).

In 1998, expression of interleukin-18 (IL-18) has been proposed to be implicated in the pathogenesis of murine contact hypersensitivity (Xu et al., 1998). Xu et al. used a murine model of contact hypersensitivity with oxazolone as contact allergen and showed an induction of IL-18 expression in skin lesions. Highest upregulation was found 24 hours after challenge with the allergen, then IL-18 expression declined gradually

A further report on the ability of IL-18 to induce a DTH response independently of IL-18 was published by Kitching et al., 2000. However, the role of IL-18 remained unclear since IL-18 was also reported to be itself a potentially effective therapy for atopic dermatitis patients (Habu et al., 2001), which was in line with several clinical trials suggesting that IFN-γ improves atopic dermatitis symptoms (Reinhold et al., 1990; Hanifin et al., 1993). EP110969 A, EP0974600 A, and EP0864585 A concern IL-18BP or anti IL-18mAb, or IL18 receptor proteins for use in the therapy of allergy, inflammation or autoimmune diseases.

## SUMMARY OF THE INVENTION

The present invention is based on the finding that treatment of mice with inhibitors of IL-18 in a model of type IV hypersensitivity results in an attenuation of the hypersensitivity reaction in the animal as compared to control animals. The invention therefore relates to the use of an IL-18 inhibitor according to claim 1 for the manufacture of a medicament for treatment and/or prevention of hypersensitivity disorders. The use of combinations of an IL-18 inhibitor with an interferon and/or an inhibitor of TNF and/or inhibitors of inflammation and/or anti-allergic drugs are also contemplated according to the invention. In a further aspect, the invention relates to the use of an expression vector comprising the coding sequence of an IL-18 inhibitor for the treatment and/or prevention of hypersensitivity conditions. The invention further relates to the use of cells genetically engineered to express IL-18 inhibitors for the prevention and/or treatment of hypersensitivity disorders.

## BRIEF DESCRIPTION OF THE DRAWINGS

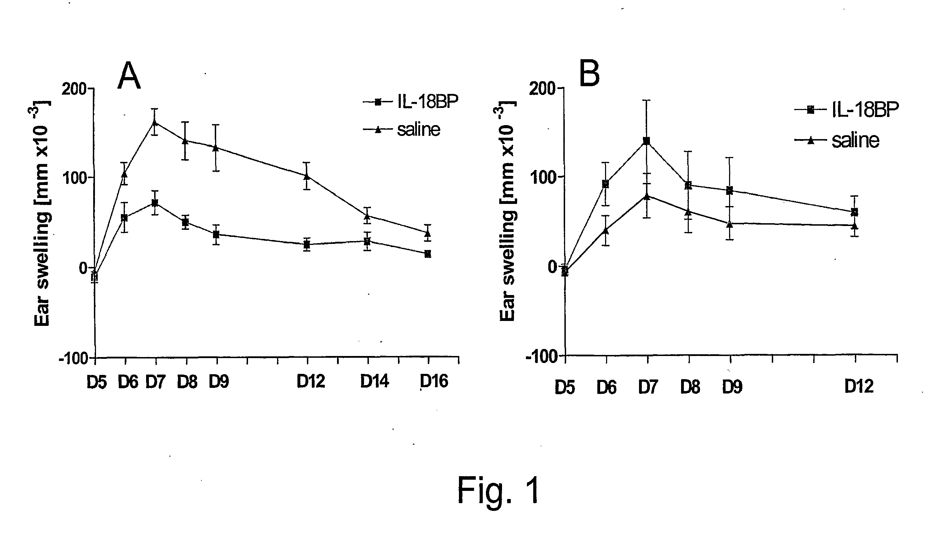

Fig. 1    shows that treatment with IL-18BP during challenge protects from contact hypersensitivity (CHS). Mice were sensitized with DNFB at the back at day 0 and challenged five days later at the ears. The ear swelling was measured daily and expressed as the increase in swelling of the DNFB challenged vs. the vehicle treated control ear. Treatment with 250 μg IL-18BP i.p. per mouse daily at days 5 to 8 markedly reduced ear swelling (A), whereas treatment at days 0 to 2 was not protective in this experimental setting (B) (n = 5 mice per group). Squares: IL18BP treated mice, Triangles: control, i.e. saline treated animals.

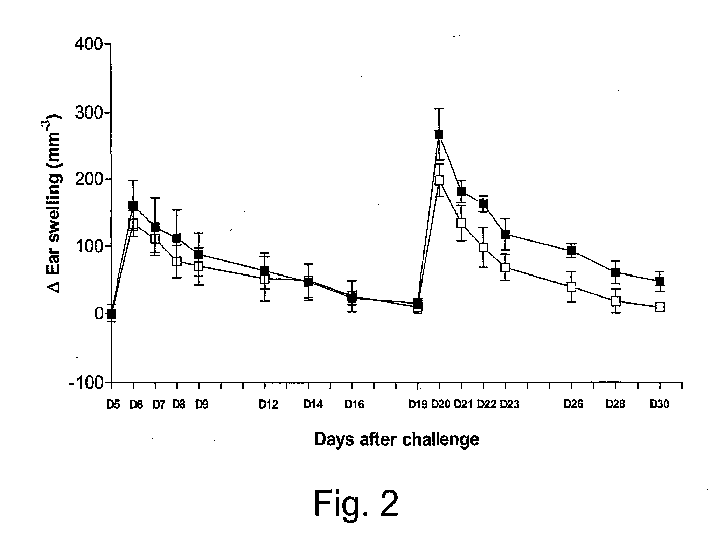

Fig. 2    shows the extent of ear swelling from day 5 to day 30 after first hapten challenge at day 5 and second challenge at day 19 in a delayed type hypersensitivity model with systemic administration of 250 μg/mouse/day IL-18BP (open squares) or vehicle (filled squares) from days 19 to 22.

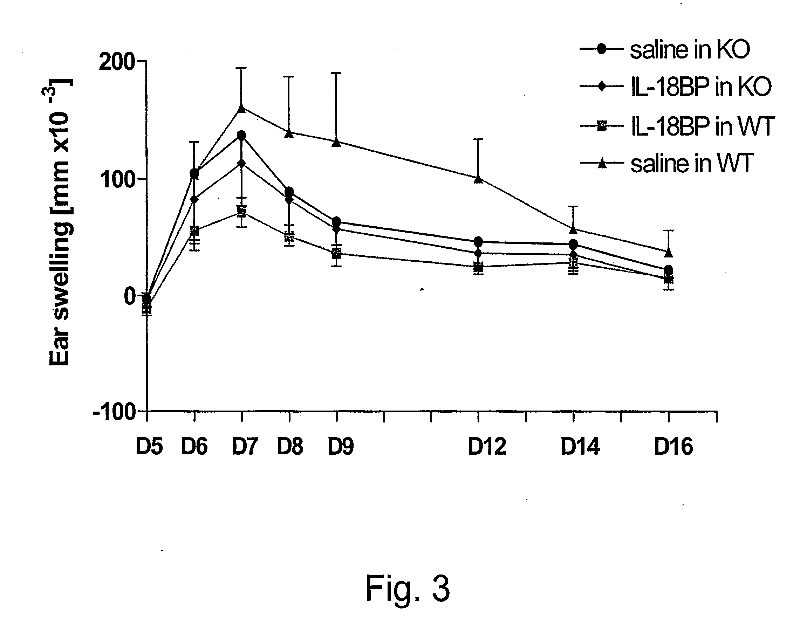

Fig. 3    shows that IL-18BP protects from CHS by neutralizing IL-18. IL-18 deficient (KO) and wild type C57BL/6 mice were compared for their ability to mount a CHS response. IL-18 deficient mice do develop CHS to DNFB, although less pronounced than wild-type mice. However, no effect of the IL-18BP treatment was observed in IL-18 deficient mice, indicating that the anti-inflammatory effect of IL-18BP in CHS was due to neutralization of IL-18. (n = 5 mice per group). Circles: Saline in IL-18 KO mice; diamonds: IL-18BP in IL-18 KO mice; squares: IL-18BP in wild type (WT) mice; triangles: saline in WT mice.

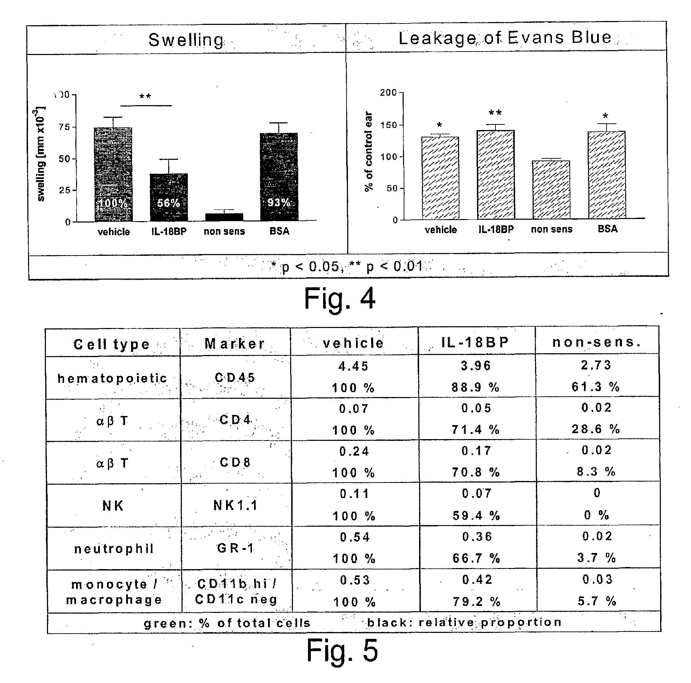

Fig. 4    shows that IL-18BP does not reduce vascular leakage during CHS. CHS was induced in C57BL/6 mice. To monitor oedema caused by the CHS reaction, Evans Blue was injected i.v. 2 h prior to challenge with DNFB. Mice were sacrificed 24h later and ears processed to extract the dye that had leaked from the vasculature and accumulated in the surrounding tissue. Vascular leakage was assessed as amount of dye per mg of dried ear tissue corrected for the concentration of Evans Blue in the serum and expressed as the ratio of challenged vs. control ear. While treatment with IL-18BP at day 4 and day 5 reduced swelling to 56% of the vehicle treated control (left panel, p < 0.01), there was no significant difference in vascular leakage between these two groups. Both groups showed significantly increased edema as compared to the non sensitized control group (p < 0.05 and p < 0.01). As a further control, mice were treated with 250μg of the irrelevant protein BSA per animal and day. These mice developed CHS like the vehicle treated control animals. (n = 10 mice per group)

Figs. 5    IL-18BP treatment reduces inflammatory infiltration of the DNFB challenged ear. CHS was induced in C57BL/6 mice as described. The animals were IL-18BP or vehicle treated at days 4 to 6. The IL-18BP treatment reduced the swelling to 58% of the vehicle control at day 7. Mice were sacrificed at day 7, challenged ears collected, pooled by group (n = 8) and enzyme digested to obtain single cell suspensions. Cells were characterized by subsequent FACS analysis gating on CD45 positive live cells. The number of αβT cells, NK cells, neutrophils and monocytes / macrophages found in the ear preparations are expressed as percentage of total cells analyzed (upper values). The reduction of these cell types after IL-18BP treatment relative to the vehicle control is given in lower figures.

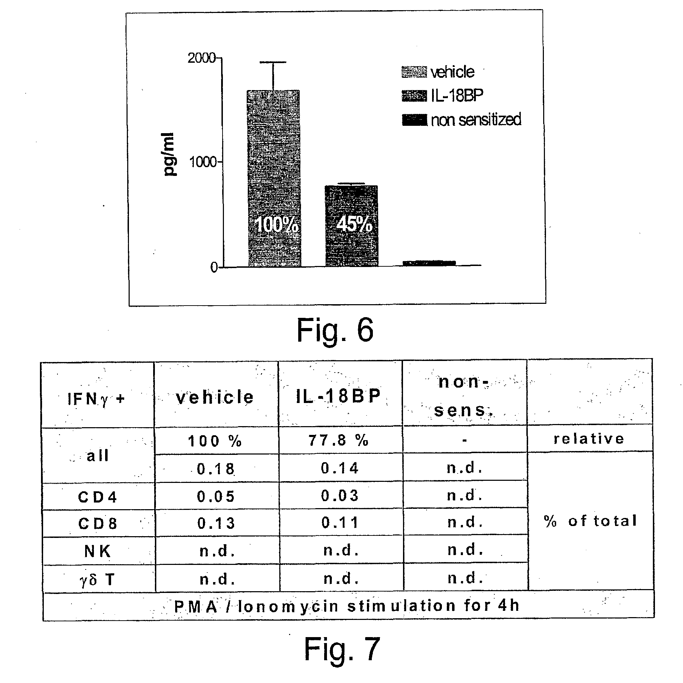

Fig. 6    shows that T cell activation is impaired upon IL-18BP treatment. Cells obtained from DNFB challenged ears were re-stimulated at $2 \times 10^5$ per well with plate bound anti-CD3 antibody. No further IL-18BP was added during the subsequent 24h culture period. IFNγ production was measured in triplicate by ELISA. The cells obtained from IL-18BP treated mice produced only 45% of the IFNγ found in the cultures of cells from vehicle treated control animals.

Fig. 7    shows that IL-18BP treatment reduces the number of IFNγ producing cells in the inflammatory infiltrate of the ear. Cell preparations from DNFB challenged ears were stimulated with 50 ng/ml PMA* and 500 ng/ml Ionomycin for 4h. Cytokine secretion was blocked by the addition of 2μg/ml brefeldin A for the last 2 h of the incubation. Cells were then subjected to multicolor immunofluorescent staining for intracellular IFNγ and surface antigens. The IL-18BP treatment reduced the total number of cells positive for IFNγ staining to 78% of the vehicle control. The IFNγ was produced by CD8 T cells and to a lesser extent by CD4 T cells. No IFNγ was detected in NK cells and αβT cells. (n.d., not detected; * Phorbol 12-Myristate 13-Acetate)

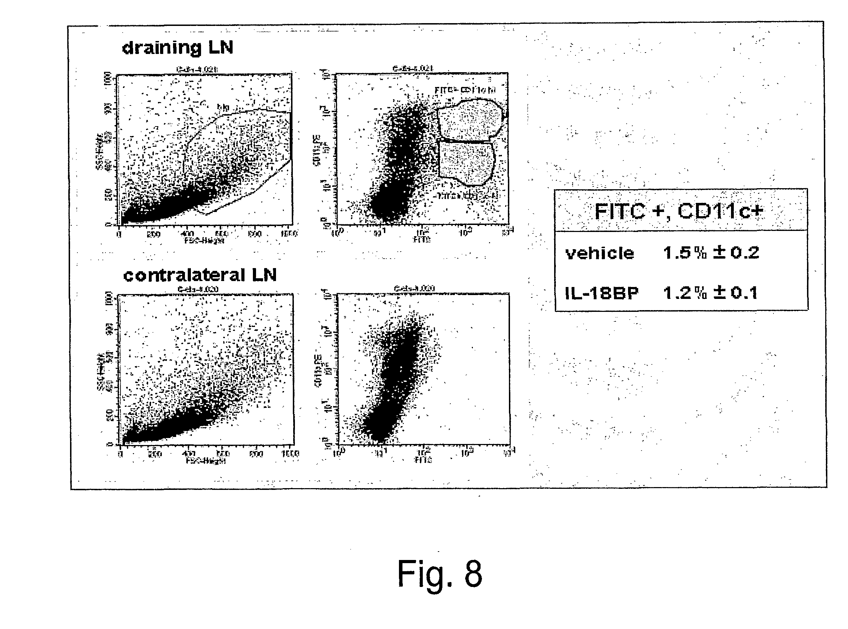

Fig. 8:    IL-18BP treatment does not impair the recruitment of Langerhans cells to the draining lymph node. Mice were painted with the hapten FITC or the vehicle acetone / dibutylphtalate (1:1) onto the right and left flank, respectively. Inguinal lymph nodes were collected 24h after the painting. Hapten conjugated Langerhans cells could be detected by FACS as FITC+, CD11 c+ cells in the lymph node draining the FITC painted flank, but not in the contralateral lymph node draining the flank painted with vehicle only. The proportion of hapten carrying Langerhans cells in the draining lymph node was 1.2% of total lymph node cells in animals treated with IL-18BP 24h and 1h prior to the painting. This did not significantly differ from the number obtained with control treated animals. (n = 5 draining lymph nodes per group)

DESCRIPTION OF THE INVENTION

The present invention is based on the finding that an IL-18 inhibitor exerted a beneficial effect on the recovery from hapten challenge in a murine model of type IV hypersensitivity.

Therefore, the invention relates to the use of an IL-18 inhibitory according to claim 1 for the manufacture of a medicament for treatment and/or prevention of hypersensitivity disorders.

Within the context of the present invention, the expressions "hypersensitivity disorder" and "allergic disorder" are used synonymously. Both terms relate to disorders or reactions caused by an inappropriate adaptive immune response. Hypersensitivity reactions are the result of normally beneficial immune responses acting inappropriately to foreign antigens, such as e.g. usually environmental macromolecules, which may lead to inflammatory reactions and tissue damage. In hypersensitivity disorders, a normally harmless stimulus, the allergen, triggers an immune response, which upon re-exposure, is re-activated to generate pathological damage.

Hypersensitivity disorders, as well as their clinical symptoms and implications have been described in detail in the "Background of the invention" and the use according to the invention relates to the hypersensitivity disorders mentioned therein.

In an embodiment of the present invention, the hypersensitivity disorder is selected from the group consisting of disorders with, type IV hypersensitivity reactions.

Disorders with type I hypersensitivity are also called immediate hypersensitivity or common allergy. The hypersensitivity is IgE-mediated. Immediate hypersensitivity (type I) reactions are due to the binding between antigen and IgE on mast cells or basophils. Within the meaning of disorders with type I hypersensitivity reactions are the atopic diseases, such as allergic rhinitis, allergic conjunctivitis, atopic dermatitis, allergic extrinsic asthma, of urticaria, systemic anaphylaxis. Anaphylaxis is severe, life-threatening allergic reaction due to type I (immediate) hypersensitivity.

Type II or cytotoxic hypersensitivity involves cytolytic actions mediated by antibody, complement, and/or cellular mechanisms. Antibody to cell-bound antigen (type II) causes cell destruction by activating complement or promoting phagocytosis. Disorders with type II hypersensitivity comprise e.g. Coombs'-positive hemolytic anemias, antibody-induced thrombocytopenic purpura, leukopenia, pemphigus, pemphigoid, Goodpasture's syndrome, and pernicious anemia. These reactions may occur in patients receiving incompatible transfusions, in hemolytic disease of the newborn, and in neonatal thrombocytopenia, and they also may play a part in multisystem hypersensitivity diseases (e.g. systemic lupus erythematosus, SLE), for instance.

Disorders with type III hypersensitivity involve reactions in which antibodies forming immune complexes with antigen. Circulating complexes activate complement, attach to red blood cells, which are then phagocytosed in the spleen, leave the circulation and trigger inflammation in tissue spaces. This reaction is called Arthus reaction. Alternatively, complexes are phagocytosed by macrophages which present antigen, release cytokines and activate B and T-cells. IgE, IgA, IgG, and IgM all form complexes with antigen. Type III reactions generally result from deposition of immune complexes in tissues, particularly the skin, joints and kidneys. Chronic immune complex nephritis accounts for most cases of glomerulonephritis in humans. According to the invention, hypersensitivity disorders of type III comprise e.g. serum sickness due to serum, drugs, or viral hepatitis antigen; SLE; rheumatoid arthritis (RA); polyarteritis; cryoglobulinemia; hypersensitivity pneumonitis; bronchopulmonary aspergillosis; acute glomerulonephritis; chronic membranoproliferative glomerulonephritis; and associated renal disease.

Disorders with type IV hypersensitivity involve cell-mediated reactions and generally take 12 or more hours to develop. Type IV hypersensitivity disorders may involve inflammation, and chronic inflammatory disease may be the result. Type IV hypersensitivity is also called delayed-type hypersensitivity or DTH. Once T-cells have been sensitised by primary exposure, secondary challenge is followed by a delayed-type hypersensitivity reaction. This reaction is a local inflammatory response, which sometimes takes 2-3 days to develop clinically.

In a preferred embodiment of the invention, the hypersensitivity disorder is delayed type hypersensitivity. Thus, the invention relates to all kinds of clinical conditions in which type IV reactions are important, such as to delayed type contact hypersensitivity, dermatitis, contact dermatitis, hypersensitivity pneumonitis, allograft rejection, granulomas due to intracellular organisms, some forms of drug sensitivity, thyroiditis, and encephalomyelitis after rabies vaccination.

DTH may result from the normal cell-mediated immune response to infection with viruses, fungi and certain bacteria, notably Mycobacterium tuberculosis and Mycobacterium leprae. Further external agents eliciting DTH can be plant, animal, insect or reptilian secretions, chemical or biochemical antigens. They can be derived from synthetic or natural sources. Various types of fibers, fabrics, such as latex used in surgical gloves, can give rise to T-cell mediated hypersensitivity reaction in certain individuals. The offending external agents can be water-borne agents such as dissolved salts and minerals, encountered for example in environmental, mining, metallurgical and chemical manufacturing operations.

In another preferred embodiment of the invention, the hypersensitivity disorder is contact dermatitis or contact hypersensitivity. Contact dermatitis, also a type IV reaction, is a reaction to occupational and other antigens. Agents eliciting contact dermatitis are usually of comparatively low molecular weight (< 1 kD) and not immunogenic on their own, instead, they are highly reactive molecules that bind covalently to skin or tissue proteins. The sensitising chemical is called a hapten and the host protein it combines with is called the carrier. Many haptens eliciting contact dermatitis are known. In order to find out whether an individual will develop contact dermatitis against a given sensitizer, a suspected contact sensitiser is applied on the patient's back and covered for 48 hours. The reaction site is inspected after 2 and 24 hours. In a positive response there is inflammation and induration at the test site.

Delayed type hypersensitivity is also a key mechanism determining the rejection of transplanted test organs, and therefore the invention further relates to the use of an IL-18 inhibitor for the prevention of graft rejection.

The term "inhibitor of IL-18" within the context of this invention refers to any molecule according to claim 1 modulating IL-18 production and/or action in such a way that IL-18 production and/or action is attenuated, reduced, or partially, substantially or completely prevented or blocked. The term "IL-18 inhibitor" is meant to encompass inhibitors of IL-18 production as well as of inhibitors of IL-18 action.

An inhibitor of production can be any molecule negatively affecting the synthesis, processing or maturation of IL-18. The inhibitors considered according to the invention can be, for example, suppressors of gene expression of the interleukin IL-18, antisense mRNAs reducing or preventing the transcription of the IL-18 mRNA or leading to degradation of the mRNA, proteins impairing correct folding, or partially or substantially preventing secretion of IL-18, proteases degrading IL-18, once it has been synthesized, inhibitors of proteases cleaving pro-IL-18 in order to generate mature IL-18, such as inhibitors of caspase-1.

An inhibitor of IL-18 action can be an IL-18 antagonist, for example. Antagonists can either bind to or sequester the IL-18 molecule itself with sufficient affinity and specificity to partially or substantially neutralize the IL-18 or IL-18 binding site(s) responsible for IL-18 binding to its ligands (like, e.g. to its receptors). An antagonist may also inhibit the IL-18 signaling pathway, which is activated within the cells upon IL-18/receptor binding.

Inhibitors of IL-18 action may also be soluble IL-18 receptors or molecules mimicking the receptors, or agents blocking the IL-18 receptors, or IL-18 antibodies, such as polyclonal or monoclonal antibodies, or any other agent or molecule preventing the binding of IL-18 to its targets, thus diminishing or preventing triggering of the intra-or extracellular reactions mediated by IL-18.

In a preferred embodiment of the present invention, the inhibitor of IL-18 is as defined in claim 1.

The term "IL-18 binding proteins" is used herein synonymously with "IL-18 binding protein" or "IL18BP". It comprises IL-18 binding proteins as defined in WO 99/09063 or in Novick et al., 1999, including splice variants and/or isoforms of IL-18 binding proteins, as defined in Kim et al., 2000, which bind to IL-18. In particular, human isoforms a and c of IL-18BP are useful in accordance with the presence invention. The proteins useful according to the present invention may be glycosylated or non-glycosylated, they may be derived from natural sources, such as urine, or they may preferably be produced recombinantly. Recombinant expression may be carried out in prokaryotic expression systems like E. coli, or in eukaryotic, and preferably in mammalian, expression systems.

As used herein the term "muteins" refers to analogs of an IL-18BP, or analogs of a viral IL-18BP, in which one or more of the amino acid residues of a natural IL-18BP or viral IL-18BP are replaced by different amino acid residues, or are deleted, or one or more amino acid residues are added to the natural sequence of an IL-18BP, or a viral IL-18BP, without changing considerably the activity of the resulting products as compared with the wild type IL-18BP or viral IL-18BP. These muteins are prepared by known synthesis and/or by site-directed mutagenesis techniques, or any other known technique suitable therefor.

Muteins include proteins encoded by a nucleic acid, such as DNA or RNA, which hybridizes to DNA or RNA, which encodes an IL-18BP or encodes a viral IL-18BP, in accordance with the present invention, under stringent conditions. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §§6.3 and 6.4 (1987, 1992), and Sambrook et al., supra. Without limitation, examples of stringent conditions include washing conditions 12-20°C below the calculated Tm of the hybrid under study in, e.g., 2 x SSC and 0.5% SDS for 5 minutes, 2 x SSC and 0.1% SDS for 15 minutes; 0.1 x SSC and 0.5% SDS at 37°C for 30-60 minutes and then, a 0.1 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, it is preferable to use tetramethyl ammonium chloride (TMAC) instead of SSC. See Ausubel, supra.

Identity reflects a relationship between two or more polypeptide sequences or two or more polynucleotide sequences, determined by comparing the sequences. In general, identity refers to an exact nucleotide to nucleotide or amino acid to amino acid correspondence of the two polynucleotides or two polypeptide sequences, respectively, over the length of the sequences being compared.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Methods for comparing the identity and homology of two or more sequences are well known in the art. Thus for instance, programs available in the Wisconsin Sequence Analysis Package, version 9.1 (Devereux J et al., 1984), for example the programs BESTFIT and GAP, may be used to determine the % identity between two polynucleotides and the % identity and the % homology between two polypeptide sequences. BESTFIT uses the "local homology" algorithm of Smith and Waterman (1981) and finds the best single region of similarity between two sequences. Other programs for determining identity and/or similarity between sequences are also known in the art, for instance the BLAST family of programs (Altschul S F et al, 1990, Altschul S F et al, 1997, accessible through the home page of the NCBI at www.ncbi.nlm.nih.gov) and FASTA (Pearson W R, 1990; Pearson 1988).

Any such mutein preferably has a sequence of amino acids sufficiently duplicative of that of an IL-18BP, or sufficiently duplicative of a viral IL-18BP, such as to have substantially similar activity to IL-18BP. One activity of IL-18BP is its capability of binding IL-18. As long as the mutein has substantial binding activity to IL-18, it can be used in the purification of IL-18, such as by means of affinity chromatography, and thus can be considered to have substantially similar activity to IL-18BP. Thus, it can be determined whether any given mutein has substantially the same activity as IL-18BP by means of routine experimentation comprising subjecting such a mutein, e.g., to a simple sandwich competition assay to determine whether or not it binds to an appropriately labeled IL-18, such as radioimmunoassay or ELISA assay.

Any such mutein has at least 40% identity or homology with the sequence of either an IL-18BP or a virally-encoded IL-18BP homologue, as defined in WO 99/09063. More preferably, it has at least 50%, at least 60%, at least 70%, at least 80% or, most preferably, at least 90% identity or homology thereto.

Muteins of IL-18BP polypeptides or muteins of viral IL-18BPs or nucleic acid coding therefor, include a finite set of substantially corresponding sequences as substitution peptides or polynucleotides which can be routinely obtained by one of ordinary skill in the art, without undue experimentation, based on the teachings and guidance presented herein.

Preferred changes for muteins are what are known as "conservative" substitutions. Conservative amino acid substitutions of IL-18BP polypeptides or proteins or viral IL-18BPs, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g., under thirty, and preferably under ten, and do not remove or displace amino acids which are critical to a functional conformation, e.g., cysteine residues. Proteins and muteins produced by such deletions and/or insertions come within the purview of the present invention.

Preferably, the synonymous amino acid groups are those defined in Table 1. More preferably, the synonymous amino acid groups are those defined in Table 2; and most preferably the synonymous amino acid groups are those defined in Table 3.

TABLE 1

Preferred Groups of Synonymous Amino Acids

| Amino Acid | Synonymous Group |
|---|---|
| Ser | Ser, Thr, Gly, Asn |
| Arg | Arg, Gln, Lys, Glu, His |
| Leu | Iie, Phe, Tyr, Met, Val, Leu |
| Pro | Gly, Ala, Thr, Pro |
| Thr | Pro, Ser, Ala, Gly, His, Gin, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gin, Thr, Arg, His |
| Gln | Glu, Lys, Asn, His, Thr, Arg, Gin |
| Asn | Gin, Asp, Ser, Asn |
| Lys | Glu, Gln, His, Arg, Lys |
| Asp | Glu, Asn, Asp |
| Glu | Asp, Lys, Asn, Gin, His, Arg, Glu |
| Met | Phe, Ile, Val, Leu, Met |
| Trp | Trp |

TABLE 2

More Preferred Groups of Synonymous Amino Acids

| Amino Acid | Synonymous Group |
|---|---|
| Ser | Ser |
| Arg | His, Lys, Arg |
| Leu | Leu, lie, Phe, Met |
| Pro | Ala, Pro |
| Thr | Thr |
| Ala | Pro, Ala |
| Val | Val, Met, Ile |
| Gly | Gly |
| Ile | lie, Met, Phe, Val, Leu |
| Phe | Met, Tyr, Ile, Leu, Phe |
| Tyr | Phe, Tyr |
| Cys | Cys, Ser |
| His | His, Gin, Arg |
| Gin | Glu, Gin, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

TABLE 3

Most Preferred Groups of Synonymous Amino Acids

| Amino Acid | Synonymous Group |
|---|---|
| Ser | Ser |
| Arg | Arg |
| Leu | Leu, lie, Met |
| Pro | Pro |
| Thr | Thr |
| Ala | Ala |
| Val | Val |
| Gly | Gly |
| lie | lie, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining muteins of IL-18BP polypeptides or proteins, or muteins of viral IL-18BPs, include any known method steps, such as presented in US patents 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

The term "fused protein" refers to a polypeptide comprising an IL-18BP, or a viral IL-18BP, or a mutein or fragment thereof, fused with another protein, which, e.g., has an extended residence time in body fluids. An IL-18BP or a viral IL-18BP, may thus be fused to another protein, polypeptide or the like, e.g., an immunoglobulin or a fragment thereof.

"Functional derivatives" as used herein cover derivatives of IL-18BPs or a viral IL-18BP, and their muteins and fused proteins, which may be prepared from the functional groups which occur as side chains on the residues or the N- or C-terminal groups, by means known in the art, and are included in the invention as long as they remain pharmaceutically acceptable, i.e. they do not destroy the activity of the protein which is substantially similar to the activity of IL-18BP, or viral IL-18BPs, and do not confer toxic properties on compositions containing it.

These derivatives include polyethylene glycol side-chains, which may mask antigenic sites and extend the residence of an IL-18BP or a viral IL-18BP in body fluids. Also disclosed herein are derivatives which include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

As "active fractions" of an IL-18BP, or a viral IL-18BP, fused proteins, the present invention covers any fragment or precursors of the polypeptide chain of the protein molecule alone or together with associated molecules or residues linked thereto, e.g., sugar or phosphate residues, or aggregates of the protein molecule or the sugar residues by themselves, provided said fraction has substantially similar activity to IL-18BP.

The term "salts" herein refers to both salts of carboxyl groups and to acid addition salts of amino groups of IL-18 inhibitor molecule, or analogs thereof. Salts of a carboxyl group may be formed by means known in the art and include inorganic salts, for example, sodium, calcium, ammonium, ferric or zinc salts, and salts with organic bases as those formed, for example, with amines, such as triethanolamine, arginine or lysine, piperidine, procaine. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of the IL-18 inhibitor relevant to the present invention, such as exerting a beneficial effect on DHT, for example.

In a further preferred embodiment, the inhibitor of IL-18 is an IL-18 antibody. Anti-IL-18 antibodies may be polyclonal or monoclonal, chimeric, humanized, or even fully human. Recombinant antibodies and fragments thereof are characterized by high affinity binding to IL-18 *in vivo* and low toxicity. The antibodies are characterized by their ability to treat patients for a period sufficient to have good to excellent regression or alleviation of the pathogenic condition or any symptom or group of symptoms related to a pathogenic condition, and a low toxicity.

Neutralizing antibodies are readily raised in animals such as rabbits, goat or mice by immunization with IL-18. Immunized mice are particularly useful for providing sources of B cells for the manufacture of hybridomas, which in turn are cultured to produce large quantities of anti-IL-18 monoclonal antibodies.

Chimeric antibodies are immunoglobulin molecules characterized by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined (Elliott et al., 1994). Humanized antibodies are immunoglobulin molecules created by genetic engineering techniques in which the murine constant regions are replaced with human counterparts while retaining the murine antigen binding regions. The resulting mouse-human chimeric antibody preferably have reduced immunogenicity and improved pharmacokinetics in humans (Knight et al., 1993).

Thus, in a further preferred embodiment, IL-18 antibody is a humanized IL-18 antibody. Preferred examples of humanized anti-IL-18 antibodies are described in the European Patent Application EP 0 974 600, for example.

In yet a further preferred embodiment, the IL-18 antibody is fully human. The technology for producing human antibodies is described in detail e.g. in WO00/76310, WO99/53049, US 6,162,963 or AU5336100. Fully human antibodies are recombinant antibodies, preferably produced in transgenic animals, e.g. xenomice, comprising all or parts of functional human immunoglobulin loci.

In a highly preferred embodiment of the present invention, the inhibitor of IL-18 is an IL-18BP as defined in claim 1.

The sequences of IL-18BP and its splice variants/isoforms can be taken from WO99/09063 or from Novick et al., 1999, as well as from Kim et al., 2000.

Functional derivatives of IL-18BP may be conjugated to polymers in order to improve the properties of the protein, such as the stability, half-life, bioavailability, tolerance by the human body, or immunogenicity. To achieve this goal, IL18-BP may be linked e.g. to Polyethlyenglycol (PEG). PEGylation may be carried out by known methods, described in WO 92/13095, for example.

Therefore, in a preferred embodiment, the functional derivative comprises at least one moiety attached to one or more functional groups, which occur as one or more side chains on the amino acid residues. An embodiment in which the moiety is a polyethylene glycol (PEG) moiety is highly preferred.

In a further preferred embodiment of the invention, the inhibitor of IL-18 comprises an immunoglobulin fusion, i.e. the inhibitor of IL-18 is a fused protein comprising all or part of an IL-18 binding protein, which is fused to all or a portion of an immunoglobulin. Methods for making immunoglobulin fusion proteins are well known in the art, such as the ones described in WO 01/03737, for example. The person skilled in the art will understand that the resulting fusion protein of the invention retains the biological activity of IL-18BP, in particular the binding to IL-18. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gin-Phe-Met introduced between the IL-18BP sequence and the immunoglobulin sequence. The resulting fusion protein has improved properties, such as an extended residence time in body fluids (half-life), increased specific activity, increased expression level, or the purification of the fusion protein is facilitated.

In a preferred embodiment, IL-18BP is fused to the constant region of an Ig molecule. Preferably, it is fused to heavy chain regions, like the CH2 and CH3 domains of human IgG1, for example. The generation of specific fusion proteins comprising IL-18BP and a portion of an immunoglobulin are described in example 11 of WO 99/09063, for example. Other isoforms of Ig molecules are also suitable for the generation of fusion proteins according to the present invention, such as isoforms $IgG_2$ or $IgG_4$, or other Ig classes, like IgM or IgA, for example. Fusion proteins may be monomeric or multimeric, hetero- or homomultimeric.

Interferons are predominantly known for inhibitory effects on viral replication and cellular proliferation. Interferon-γ, for example, plays an important role in promoting immune and inflammatory responses. Interferon β (IFN-β, an interferon type I), is said to play an anti-inflammatory role.

The invention therefore also relates to the use of a combination of an inhibitor of IL-18 and an interferon in the manufacture of a medicament for the treatment of hypersensitivity disorders.

Interferons may also be conjugated to polymers in order to improve the stability of the proteins. A conjugate between Interferon β and the polyol Polyethlyenglycol (PEG) has been described in WO99/55377, for instance.

In another embodiment, the interferon is Interferon-β (IFN-β), and more preferably IFN-β 1a.

The inhibitor of IL-18 production and/or action is preferably used simultaneously, sequentially, or separately with the interferon.

In yet a further embodiment of the invention, an inhibitor of IL-18 is used in combination with a TNF antagonist. TNF antagonists exert their activity in several ways. First, antagonists can bind to or sequester the TNF molecule itself with sufficient affinity and specificity to partially or substantially neutralize the TNF epitope or epitopes responsible for TNF receptor binding (hereinafter termed "sequestering antagonists"). A sequestering antagonist may be, for example, an antibody directed against TNF.

Alternatively, TNF antagonists can inhibit the TNF signaling pathway activated by the cell surface receptor after TNF binding (hereinafter termed "signaling antagonists"). Both groups of antagonists are useful, either alone or together, in combination with an IL-18 inhibitor, in the therapy of hypersensitivity disorders.

TNF antagonists are easily identified and evaluated by routine screening of candidates for their effect on the activity of native TNF on susceptible cell lines in vitro, for example human B cells, in which TNF causes proliferation and immunoglobulin secretion. The assay contains TNF formulation at varying dilutions of candidate antagonist, e.g. from 0.1 to 100 times the molar amount of TNF used in the assay, and controls with no TNF or only antagonist (Tucci et al., 1992).

Sequestering antagonists are the preferred TNF antagonists to be used according to the present invention. Amongst sequestering antagonists, those polypeptides that bind TNF with high affinity and possess low immunogenicity are preferred. Soluble TNF receptor molecules and neutralizing antibodies to TNF are particularly preferred. For example, soluble TNF-RI and TNF-RII are useful in the present invention. Truncated forms of these receptors, comprising the extracellular domains of the receptors or functional portions thereof, are more particularly preferred antagonists according to the present invention. Truncated soluble TNF type-I and type-II receptors are described in EP914431, for example.

Truncated forms of the TNF receptors are soluble and have been detected in urine and serum as 30 kDa and 40 kDa TNF inhibitory binding proteins, which are called TBPI and TBPII, respectively (Engelmann et al., 1990). The simultaneous, sequential, or separate use of the IL-18 inhibitor with the TNF antagonist and /or an Interferon is preferred, according to the invention.

According to the invention, TBP I and TBPII are preferred TNF antagonists to be used in combination with an IL-18 inhibitor. Derivatives, fragments, regions and biologically active portions of the receptor molecules functionally resemble the receptor molecules that can also be used in the present invention. Such biologically active equivalent or derivative of the receptor molecule refers to the portion of the polypeptide, or of the sequence encoding the receptor molecule, that is of sufficient size and able to bind TNF with such an affinity that the interaction with the membrane-bound TNF receptor is inhibited or blocked.

In a further preferred embodiment, human soluble TNF-RI (TBPI) is the TNF antagonist to be used according to the invention. The natural and recombinant soluble TNF receptor molecules and methods of their production have been described in the European Patents EP 308 378, EP 398 327 and EP 433 900.

The IL-18 inhibitor can be used simultaneously, sequentially or separately with the TNF inhibitor.

In a further preferred embodiment of the invention, the medicament further comprises an anti-inflammatory agent, such as an NSAID (nonsteroidal anti-inflammatory drugs). In a preferred embodiment, a COX-inhibitor, and most preferably a COX-2 inhibitor, is used in combination with an IL-18 inhibitor. COX-inhibitors are known in the art. Specific COX-2 inhibitors are disclosed in WO 01/00229, for example. The active components may be used simultaneously, sequentially, or separately.

Hypersensitivity reactions are frequently being treated with anti-allergic drugs such as antihistamines, cromolyn, glucocorticoids or sympathomimetics. Therefore, the present invention further relates to combination therapy comprising an inhibitor of IL-18 and an anti-allergic drug. The use of an antihistamine and/or cromolyn and/or a glucocorticoid and/or a sympathomimetic for separate, sequential or simultaneous use with an inhibitor of IL-18 is preferred in accordance with the present invention.

In a further preferred embodiment of the present invention, the inhibitor of IL-18 is used in an amount of about 0.0001 to 1000 mg/kg of body weight, or about 0.001 to 100 mg/kg of body weight or about 0.01 to 10 mg/kg of body weight or about 0.1 to 5 mg/kg or about 1 to 3 mg/kg of body weight

The IL-18 inhibitor according to the invention is preferably administered topically, i.e. locally. For contact dermatitis, for example, the IL-18 inhibitor may be administered directly onto the affected area of the skin.

In another embodiment of the invention, the IL-18 inhibitor is administered systemically, and preferably subcutaneously or intramuscularly.

The invention further relates to the use of an expression vector comprising the coding sequence of an inhibitor of IL-18 in the preparation of a medicament for the prevention and/or treatment of hypersensitivity disorders. Thus, a gene therapy approach is considered in order to deliver the IL-18 inhibitor to the site where it is required. In order to treat and/or prevent a hypersensitivity disorder, the gene therapy vector comprising the sequence of an inhibitor of IL-18 production and/or action may be injected directly into the diseased tissue, for example, thus avoiding problems involved in systemic administration of gene therapy vectors, like dilution of the vectors, reaching and targetting of of the target cells or tissues, and of side effects.

The use of a vector for inducing and/or enhancing the endogenous production of an inhibitor of IL-18 in a cell normally silent for expression of an IL-18 inhibitor, or which expresses amounts of the inhibitor which are not sufficient, are also contemplated according to the invention. The vector may comprise regulatory sequences functional in the cells desired to express the inhibitor or IL-18. Such regulatory sequences may be promoters or enhancers, for example. The regulatory sequence may then be introduced into the right locus of the genome by homologous recombination, thus operably linking the regulatory sequence with the gene, the expression of which is required to be induced or enhanced. The technology is usually referred to as "Endogenous Gene Activation" (EGA), and it is described e.g. in WO 91/09955.

It will be understood by the person skilled in the art that it is also possible to shut down IL-18 expression directly, without using an inhibitor of IL-18, with the same technique. To do that, a negative regulation element, like e.g. a silencing element, may be introduced into the gene locus of IL-18, thus leading to down-regulation or prevention of IL-18 expression. The person skilled in the art will understand that such down-regulation or silencing of IL-18 expression has the same effect as the use of an IL-18 inhibitor in order to prevent and/or treat disease.

The invention further relates to the use of a cell that has been genetically modified to produce an inhibitor of IL-18 in the manufacture of a medicament for the treatment and/or prevention of hypersensitivity disorders.

The invention further relates to pharmaceutical compositions, particularly useful for prevention and/or treatment of hypersensitivity disorders, which comprise a therapeutically effective amount of an inhibitor of IL-18 and/or a therapeutically effective amount of an interferon and/or a pharmaceutical effective amount of a TNF inhibitor and/or a pharmaceutically effective amount of an anti-inflammatory agent and/or a pharmaceutically effective amount of an anti-allergic agent, in particular an antihistamine.

IL-18BP and its fused proteins, functional derivatives, active fractions according to claim 1 are the preferred active ingredients of the pharmaceutical compositions.

The interferon comprised in the pharmaceutical composition is preferably IFN-β.

In yet another preferred embodiment, the pharmaceutical composition comprises therapeutically effective amounts of an inhibitor of TNF alpha. The pharmaceutical composition according to the invention may further comprise one or more COX-inhibitors.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g. in slow release formulations), intramuscular, intraperitoneal, intravenous, subcutaneous, oral, intracranial, epidural, topical, rectal, and intranasal routes. Any other therapeutically efficacious route of administration can be used, for example absorption through epithelial or endothelial tissues or by gene therapy wherein a DNA molecule encoding the active agent is administered to the patient (e.g. via a vector) which causes the active agent to be expressed and secreted in vivo. In addition, the protein(s) according to the invention can be administered together with other components of biologically active agents such as pharmaceutically acceptable surfactants, excipients, carriers, diluents and vehicles.

For parenteral (e.g. intravenous, subcutaneous, intramuscular) administration, the active protein(s) can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the invention can also be ameliorated by using conjugation procedures which increase the half-life of the molecule in the human body, for example linking the molecule to polyethylenglycol, as described in the PCT Patent Application WO 92/13095.

The therapeutically effective amounts of the active protein(s) will be a function of many variables, including the type of antagonist, the affinity of the antagonist for IL-18, any residual cytotoxic activity exhibited by the antagonists, the route of administration, the clinical condition of the patient (including the desirability of maintaining a non-toxic level of endogenous IL-18 activity).

A "therapeutically effective amount" is such that when administered, the IL-18 inhibitor results in inhibition of the biological activity of IL-18. The dosage administered, as single or multiple doses, to an individual will vary depending upon a variety of factors, including IL-18 inhibitor pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the inhibition of IL-18 in an individual.

According to the invention, the inhibitor of IL-18 is used in an amount of about 0.001 to 100 mg/kg or about 0.01 to 10 mg/kg or body weight, or about 0. 1 to 5 mg/kg of body weight or about 1 to 3 mg/kg of body weight or about 2 mg/kg of body weight.

The route of administration which is preferred according to the invention is administration by subcutaneous route. Intramuscular administration is further preferred according to the invention. In order to administer the IL-18 inhibitor directly to the place of its action, it is also preferred to administer it topically.

In further preferred embodiments, the inhibitor of IL-18 is administered daily or every other day.

The daily doses are usually given in divided doses or in sustained release form effective to obtain the desired results. Second or subsequent administrations can be performed at a dosage which is the same, less than or greater than the initial or previous dose administered to the individual. A second or subsequent administration can be administered during or prior to onset of the disease.

According to the invention, the IL-18 inhibitor can be administered prophylactically or therapeutically to an individual prior to, simultaneously or sequentially with other therapeutic regimens or agents (e.g. multiple drug regimens), in a therapeutically effective amount, in particular with an interferon and/or a TNF inhibitor and/or another anti-inflammatory agent, such as a COX inhibitor and/or an anti-allergic agent. Active agents that are administered simultaneously with other therapeutic agents can be administered in the same or different compositions.

The invention further relates to a method for the preparation of a pharmaceutical composition comprising admixing an effective amount of an IL-18 inhibitor and/or an interferon and/or a TNF antagonist and/or a COX inhibitor with a pharmaceutically acceptable carrier.

The invention further relates to a method of treatment of hypersensitivity disorders, comprising administering a pharmaceutically effective amount of an IL-18 inhibitor to a patient in need thereof.

EXAMPLES

**Example 1:** IL-18BP treatment decreases contact hypersensitivity

Methods

Murine models of experimentally induced contact hypersensitivity (CHS) were used in all examples below. The extent of CHS is measured by ear swelling in response to a locally applied sensitizer.

The mouse ear-swelling test that was used to generate the data presented in Figs. 1 to 3 (see below) has been described in detail (Garrigue et al., 1994). Briefly, mice were sensitized topically by applying 25 μl of 0.5% 2,4-dinitrofluorobenzene (DNFB; Sigma Chemical Co.) solution in acetone/olive oil (4:1) to the shaved abdomen (day 0). Five days later, 20 μl of 0.2% DNFB in the same vehicle was applied to the right ears, and vehicle alone to the left ears.

Either mice received daily, from day 5 to day 8, either 250 μg/mouse/day of rhIL-18BP (recombinant human

IL-18BP) or saline in the control group intraperitoneally (i.p.) (Fig. 1 A), or they received IL-18BP at days 0 to 2 (Fig. 1B).

Ear thickness was measured with a dial thickness gauge (Mitutoyo Corp., Kawasaki, Japan), and ear swelling was estimated by subtracting the pre-challenge from the post-challenge value, and by further subtracting any swelling detected in the vehicle-challenged contralateral ear.

Ear swelling were measured at days 0, 5, 6, 7, 8, 9, 12, 14, 16.

Another model was used to generate the data presented in Fig. 4 to 6, see the examples below. IL-18 binding protein (IL-18BP) was used to neutralize IL-18 during experimentally induced contact hypersensitivity (CHS) to 2,4-Dinitrofluorobenzene (DNFB). The experimental setup was as follows:

1. <u>Day 0: Sensitisation</u>
25$\mu$l DNFB (0.5% in acetone / olive oil (4/1) as vehicle) on shaved back
2. <u>Day 5: Challenge</u>
5$\mu$l DNFB (0.2%) each on dorsal and ventral side of the right ear 5$\mu$l vehicle each on dorsal and ventral side of the left ear
3. <u>Day 6 ff: readout</u>
Monitor ear thickness as a measure of inflammation
Express score as increase in swelling [$\mu$m] of challenged vs. control ear
4. <u>Day 6 or 7: process ears for analysis</u>
In this model, the swelling peaks between day 6 and day 7. IL-18 was neutralized by daily injections of 250$\mu$g IL-18BP (in saline) per animal, either during sensitisation at day 0, day 1 and day 2, or during challenge at day 4, day 5 and day 6.

<u>Results</u>

Contact hypersensitivity responses (CHS) are hapten-specific skin inflammations mediated by T cells. Most haptens give rise to an oligoclonal T-cell response consisting mainly of CD8 [+] effector T cells, whereas CD4 [+] T cells have a downregulatory role in the CHS response (Bour, H., et al. 1995; Grabbe et al., 1998). To test the role of IL-18 in CHS, mice were epicutaneously sensitized with DNFB, and then challenged 5 days later by hapten application to ear skin. Concomitantly with the hapten challenge, mice were injected i.p. with either 250 $\mu$g/mouse/day rhIL-18BP or with saline. IL-18BP, or saline as control, was administered daily for 3 days, at days 5 to 8 after the first DNFB sensitization (day 0). DNFB challenge at day 5 induced significant ear swelling in both groups (Fig. 1 A). The extent of swelling in mice treated with saline (triangles) was much more pronounced than in mice treated with IL-18BP (squares), and went back to almost normal state already at day 9.

The change of ear swelling observed with IL-18BP treatment at days 0 to 2 was not statistically significant (Fig. 1 B). The timing of IL-18BP administration seems to be an important factor in order to obtain a beneficial effect of IL-18BP treatment.

<u>Conclusion: ,</u>

In this established murine model of contact hypersensitivity/contact dermatitis, a three day treatment with an inhibitor of IL-18 had a significant beneficial effect on the extent of swelling/inflammation elicited by treatment with a hapten.

**Example 2:** IL-18BP treatment decreases contact hypersensitivity after a second challenge

<u>Methods</u>

C57BL/6 Mice were sensitized by epicutaneous application of 25 $\mu$l of 0.5% DNFB solution on the shaved abdomen (day 0). Mice received a first challenge with 20 $\mu$l of 0.2% DNFB on the ears at day 5. At day 19, mice received a second challenge with DNFB. Ear swelling was measured at days 0 (hapten sensitization), 5 (1st hapten challenge), 6, 7, 8, 9, 12, 14, 16, 19 (2nd hapten challenge), 20, 21, 22, 23, 26, 28, 30. Mean values with SD for each group are presented in Fig. 2. Mice were treated daily either with 250 $\mu$g/mouse/day IL-18BP i.p. (n = 5; Fig. 2 open squares) or with saline (n = 5; Fig. 2, closed squares from day 19 to day 23.

<u>Result</u>

As shown in Fig. 2, IL-18BP-treatment significantly reduced ear swelling in particular after the second challenge with hapten.

Therefore, IL-18BP therapy is particularly suitable for the therapy of hypersensitivity disorders, in which patients are usually repeatedly challenged with the same allergen and need to be treated in order to overcome the inflammatory reactions elicited by the challenges.

**Example 3:** IL-18BP protects from CHS by neurtralizing IL-18

In order to verify that the protection from swelling observed with the IL-18BP treatment was due to the neutralization of IL-18, IL-18 deficient (KO) and wild type C57BL/6 mice were compared for their ability to mount a CHS response. IL-18 deficient mice do develop CHS to DNFB, although less pronounced than wild-type mice. However, no effect of the IL-18BP treatment was observed in IL-18 deficient mice, as shown in Fig. 3, indicating that the anti-inflammatory effect of IL-18BP in CHS was due to neutralization of IL-18 (n = 5 mice per group).

**Example 4:** IL-18BP does not reduce the vascular leakage

Methods

CHS was induced in C57BL/6 mice as described above. To monitor edema caused by the CHS reaction, Evans Blue was injected i.v. 2 h prior to challenge with DNFB. Mice were sacrificed 24h later and ears processed to extract the dye that had leaked from the vasculature and accumulated in the surrounding tissue. Vascular leakage was assessed as amount of dye per mg of dried ear tissue corrected for the concentration of Evans Blue in the serum and expressed as the ratio of challenged vs. control ear. While treatment with IL-18BP at day 4 and day 5 reduced swelling to 56% of the vehicle treated control (Fig. 4, left panel, $p < 0.01$), there was no significant difference in vascular leakage between these two groups (Fig. 4, right panel). Both groups showed significantly increased oedema as compared to the non sensitized control group ($p < 0.05$ and $p < 0.01$). As a further control, mice were treated with 250 $\mu$g of the irrelevant protein BSA per animal and day. These mice developed CHS like the vehicle treated control animals (n = 10 mice per group).

*In vivo assay for vascular leakage (Evans Blue)*

Principle: Injection (iv) of Evans Blue and extraction from target tissue (ear)

Raw data to assess:

- Standard curve of Evans Blue ($OD_{620}$/ng)
- Evans Blue concentration in blood ($OD_{620}$/ml or $\mu$g/ml, respectively)
- Dried tissue weight of ears (Ipsi and contralateral, mg)
- Evans blue content in ears (Ipsi and contralateral, $OD_{620}$/$\mu$g or ng/mg, respectively)

Protocol in combination with DNFB induced CHS:

- at day 5 of experimentally induced CHS inject 100$\mu$l of Evans Blue$^\alpha$ iv retroorbitally 2h prior to challenge of mice with DNFB
- inject IL-18BP i.p. 1h prior to challenge
- at day 6 (24h after ear challenge with DNFB) measure swelling and sacrifice animals
- take blood samples and process as follows:

  ○ add 30 $\mu$l of serum to 970 $\mu$l of formamide ($\rightarrow$ 1/33 dilution)
  ○ determine OD at 620 nm ($\rightarrow$ 1 $OD_{620}$ equals 33 $OD_{620}$/ml)

- harvest ipsilateral and contralateral ear and process as follows:

  ○ dry 24h at 80°C
  ○ determine dry weight
  ○ mince and extract dye with 1 ml formamide, gently shaking 24h at 55°C

∘ filter to remove debris[β], fill cuvettes, let sit at RT for several hours to allow lipids to float to the top
∘ determine OD at 620 nm

[α]7.5 mg/ml Evans Blue ($\Sigma$ 2129) in physiological NaCl, 100 $\mu$l, iv)
[β] Sample preparation filter Whatman 5$\mu$m PTFE mesh, #6984.0350

Values to be calculated:

$$\text{Leakage:} \rightarrow \frac{Evans\,Blue\,content\,per\,dry\,tissue\,weight\left[\frac{ng}{mg}\right]}{Evans\,Blue\,concentration\,in\,serum\left[\frac{\mu g}{ml}\right]}$$

$$\text{Relative leakage:} \rightarrow \frac{100 * leakage\,experimental}{leakage\,vehicle}$$

Results:

Basically, two processes contribute to the swelling observed during the CHS reaction: The leakage of liquid from the vasculature into the surrounding tissue causing edema, and the extravasation of inflammatory cells from the blood vessels to the site of tissue damage.
Using Evans Blue as a tracker, it was demonstrated that despite the overall reduction of swelling the treatment with IL-18BP did not reduce vascular leakage (Fig. 4).

**Example 5:** IL-18BP treatment reduces the inflammatory infiltration abd IFN$\gamma$ production of the DNFB challenged ear

Methods

CHS was induced in C57BL/6 mice as described. The animals were IL-18BP or vehicle treated at days 4 to 6. The IL-18BP treatment reduced the swelling to 58% of the vehicle control at day 7. Mice were sacrificed at day 7, challenged ears collected, pooled by group (n = 8) and enzyme digested to obtain single cell suspensions. Cells were characterized by subsequent FACS analysis gating on CD45 positive live cells. The number of $\alpha\beta$T cells, NK cells, neutrophils and monocytes / macrophages found in the ear preparations are expressed as percentage of total cells analyzed. The reduction of these cell types after IL-18BP treatment relative to the vehicle control was also calculated.
For measurement of IFN$\gamma$ production, cells obtained from DNFB challenged ears were restimulated at $2 \times 10^5$ per well with plate bound anti-CD3 antibody. No further IL-18BP was added during the subsequent 24h culture period. IFN$\gamma$ production was measured in triplicate by ELISA.
Cell preparations from DNFB challenged ears were stimulated with 50 ng/ml PMA* and 500 ng/ml Ionomycin for 4h. Cytokine secretion was blocked by the addition of 2$\mu$g/ml brefeldin A for the last 2 h of the incubation. Cells were then subjected to multicolor immunofluorescent staining for intracellular IFN$\gamma$ and surface antigens. The IFN$\gamma$ was produced by CD8 T cells and to a lesser extent by CD4 T cells. No IFN$\gamma$ was detected in NK cells and $\gamma\delta$T cells. (n.d., not detected; * Phorbol 12-Myristate 13-Acetate)

*Preparation of single cell suspension*

Enzymatic digestion of mouse ears to obtain single cell suspension was based on protocols of Schuler,G. and Steinman,R.M. (1985) and Stingl et al. (1983).

1. cut ears, pool 5 ears per preparation
2. rinse with 70% ethanol
3. split with the aid of forceps
4. place dermal side down on 7.5ml of HBSS[1] at 37°C
5. add 5ml of 2.5% Trypsin [2] (10x) to obtain a final conc. of 1 %
6. incubate 35 min at 37°C

7. transfer ear halves dermal side down to a nylon sieve (cell strainer) placed in 10ml HBSS / 80%FCS on ice and gently mesh to dislodge cells from extracellular matrix
8. remove sieves with big debris
9. wash 2x with cold HBSS / 10%FCS
10. count cells

[1] Hanks Balanced Salt Solution without $Ca^{2+}$ and $Mg^{2+}$ (GIBCO# 14170.070)
[2] 2.5% Trypsin / EDTA (10×) (GIBCO#35400-027)

*FACS analysis*

1. resuspend cells in FACS staining buffer[3], all subsequent steps on ice
2. use $10^6$ cells per staining
3. add 1μg FC-Block, 10 min
4. add anti-CD45 antibody in combination with antibodies directed against markers of interest, 1 μg each , 30 min
5. wash 2x
6. acquire 0.5 x $10^6$ total events by FACS
7. analyze specific markers after gating on CD45+, live cells

[3]1 % Bovine Serum Albumin in Phosphate Buffered Saline

Results:

To examine the inflammatory infiltrate at the site of challenge, single cell suspensions prepared from naïve and challenged ears were analysed by FACS, gating on CD45+ live cells (Fig. 5). The CD45+ cells present in the naïve ear were shown to be mainly γδT cells and dendritic cells of the skin. The inflammatory infiltrate 24h after challenge was composed of CD8 and CD4 T cells, neutrophils, monocytes and NK cells, increasing the total number of CD45+ cells in the ear by about two fold. Corresponding to the reduction in swelling, IL-18BP treatment reduced the overall number of leukocytes infiltrating the site of challenge. This affected all the different cell types of the inflammatory infiltrate. The reduction amounted to between 20% and 40% depending on the cell type.

Further characterisation of the quality of the infiltrate obtained from the ears of IL-18BP treated mice revealed an impaired IFNγ production upon anti-CD3 restimulation (Fig. 6). FACS analysis showed that the IFNγ was mainly produced by CD8 T cells and to a lesser amount by CD4 T cells. Interestingly, NK cells and γδT cells did not contribute to the IFNγ production (Fig. 7).

**Example 6:** IL-18BP does not impair recruitment of Langerhans cells

Method:

Mice were painted with the hapten FITC (50μl of a 4mg/ml solution) or the vehicle acetone / dibutylphtalate (1:1) onto the right and left flank, respectively. Inguinal lymph nodes were collected 24h after the painting. Hapten conjugated Langerhans cells could be detected by FACS as FITC+, CD11 c+ cells in the lymph node draining the FITC painted flank, but not in the contralateral lymph node draining the flank painted with vehicle only. (n = 5 draining lymph nodes per group)

Results:

The migration of Langerhans cells (LC) carrying antigen to the draining lymph node is dependent on the proinflammatory cytokines IL-1β and TNFα and regulated by caspase-1 (Antonopoulos et al., 2001; Kimber et al., 1992). Accordingly, IL-18 has been implied to contribute to LC trafficking (Cumberbatch et al., 2001). Therefore, treatment with IL-18BP might impair LC recruitment and therefore reduce the immune response to DNFB during the challenging phase. To test this hypothesis, mice were painted with the hapten FITC or the vehicle onto the right and left flank, respectively. Skin draining inguinal lymph nodes were collected 24h after the painting and the number of FITC+ cells per lymph node assessed. The treatment of the animals with IL-18BP 24h and 1h prior to the painting did not change the number of hapten carrying LC present in the draining lymph node 24h after the painting (Fig. 8). Therefore, there is no major contribution of IL-18 to LC trafficking in this model.

**Example 7:** IL-18BP reduces the extent of delay type hypersensitivity in another murine model of DTH

Methods

*Delayed-type hypersensitivity*

Mice are sensitized by intravenous injection of $10^6$ BALB/c splenocytes, and challenged on day 5 with $13 \times 10^6$ BALB/c splenocytes (50 µl PBS) in the right footpads. Control left footpads receive 50 µl PBS. Right footpad swelling is calculated on different days by subtracting the prechallenge value and any swelling measured in left footpads from the postchallenge value.

For adoptive transfer experiments, cell suspensions from lymph nodes of BALB/c splenocyte-sensitized or untreated control animals are depleted of B220$^+$ and CD8$^+$ cells by incubation with rat anti-mouse B220-FITC and CD8-FITC, followed by separation in MACS columns with paramagnetic anti-FITC microbeads (Miltenyi Biotech, Auburn, California, USA). Eluted CD4$^+$ T cell-enriched preparations are injected into the tail vein of recipient mice ($2 \times 10^7$ cells/mouse). After 16 hours, mice are challenged by injecting $13 \times 10^6$ BALB/c splenocytes (without red blood cells) into the right footpads, and swelling is monitored throughout the following days.

Result

Delayed type hypersensitivity (DHT) is elicited by CD4$^+$ T cells with apparent downregulatory effects of CD8$^+$ T cells (Grabbe et al., 1998). The behaviour of CD4$^+$ T cells from mice treated with IL-18BP is studied in a DTH model. C57BL/6 animals are sensitized by intravenous injection of $10^6$ allogeneic BALB/c splenocytes. Five days later, $13 \times 10^6$ BALB/c splenocytes are injected into the right footpads, together with either 10 mg/kg recombinant human IL-18BP i.p. or vehicle. Local inflammation is measured by determining the footpad swelling at 24 hours.

REFERENCES

1. Altschul S F et al, J Mol Biol, 215, 403-410, 1990, Altschul S F et al, Nucleic Acids Res., 25:389-3402, 1997

2. Antonopoulos, C., M. Cumberbatch, R. J. Dearman, R. J. Daniel, I. Kimber, and R. W. Groves. 2001. Functional caspase-1 is required for Langerhans cell migration and optimal contact sensitization in mice. J Immunol 166:3672-3677.

3. Bour, H., et al. 1995. Major histocompatibility complex class I-restricted CD8 + T cells and class II-restricted CD4 + T cells, respectively, mediate and regulate contact sensitivity to dinitrofluorobenzene. Eur. J. Immunol. 25:3006-3010.

4. Chater, K. F. et al., in "Sixth International Symposium on Actinomycetales Biology", Akademiai Kaido, Budapest, Hungary (1986), pp. 45-54).

5. Conti, B., J. W. Jahng, C. Tinti, J. H. Son, and T. H. Joh. 1997. Induction of interferon-gamma inducing factor in the adrenal cortex. J. Biol. Chem. 272:2035-2037.

6. Cumberbatch, M., R. J. Dearman, C. Antonopoulos, R. W. Groves, and I. Kimber. 2001. Interleukin (IL)-18 induces Langerhans cell migration by a tumour necrosis factor-alpha- and IL-1beta-dependent mechanism. Immunology 102:323-330.

7. Devereux J et al, Nucleic Acids Res, 12, 387-395, 1984.

8. DiDonato, J A, Hayakawa, M, Rothwarf, D M, Zandi, E and Karin, M. (1997), Nature 388, 16514-16517.

9. Elliott, M.J., Maini, R.N., Feldmann, M., Long-Fox, A., Charles, P., Bijl, H., and Woody, J.N., 1994, Lancet 344,1125-1127.

10. Engelmann, H., D. Novick, and D. Wallach. 1990. Two tumor necrosis factor-binding proteins purified from human urine. Evidence for immunological cross-reactivity with cell surface tumor necrosis factor receptors. J. Biol. Chem. 265:1531-1536.

11. Garrigue JL, Nicolas JF, Fraginals R, Benezra C, Bour H, Schmitt D. Contact Dermatitis 1994 Apr;30(4):231-7

12. Grabbe, S., and Schwarz, T. 1998. Immunoregulatory mechanisms involved in elicitation of allergic contact hypersensitivity. Immunol. Today. 19:37-44

13. Habu et al., J. Immunol. 2001, 166: 5439-5347.

14. Hanifin et al. (1993). J. Am. Acad. Dermatol. 28: 189.

15. Kim SH, Eisenstein M, Reznikov L, Fantuzzi G, Novick D, Rubinstein M, Dinarello CA. Structural requirements of six naturally occurring isoforms of the IL-18 binding protein to inhibit IL-18. Proc Natl Acad Sci U S A 2000;97:1190-1195.

16. Kimber, I. and M. Cumberbatch. 1992. Stimulation of Langerhans cell migration by tumor necrosis factor alpha (TNF-alpha). J Invest Dermatol. 99:48S-50S.

17. Maliszewski, C. R., T. A. Sato, T. Vanden Bos, S. Waugh, S. K. Dower, J. Slack, M. P. Beckmann, and K. H. Grabstein. 1990. Cytokine receptors and B cell functions. I. Recombinant soluble receptors specifically inhibit IL-1- and IL-4-induced B cell activities in vitro. J. Immunol. 144:3028-3033.

18. Micallef, M. J., T. Ohtsuki, K. Kohno, F. Tanabe, S. Ushio, M. Namba, T. Tanimoto, K. Torigoe, M. Fujii, M. Ikeda, S. Fukuda, and M. Kurimoto. 1996. Interferon-gamma-inducing factor enhances T helper 1 cytokine production by stimulated human T cells: synergism with interleukin-12 for interferon-gamma production. Eur-J-Immunol 26:1647-51 issn: 0014-2980.

19. Nakamura K, Okamura H, Wada M, Nagata K, Tamura T. Infect Immun 1989 Feb;57(2):590-5

20. Novick, D, Kim, S-H, Fantuzzi, G, Reznikov, L, Dinarello, C, and Rubinstein, M (1999). Immunity 10, 127-136.

21. Okamura H, Nagata K, Komatsu T, Tanimoto T, Nukata Y, Tanabe F, Akita K, Torigoe K, Okura T, Fukuda S, et al. Infect Immun 1995 Oct;63(10):3966-72

22. Parnet, P, Garka, K E, Bonnert, T P, Dower, S K, and Sims, J E. (1996), J. Biol. Chem. 271, 3967-3970.

23. Reinhold et al. (1990). Lancet 335: 1282.

24. Rothe H, Jenkins NA, Copeland NG, Kolb H. J Clin Invest 1997 Feb 1;99(3):469-74

25. Schuler,G. and Steinman,R.M. (1985). Murine epidermal Langerhans cells mature into potent immunostimulatory dendritic cells in vitro. J Exp. Med. 161, 526-546.

26. Stingl,L.A., Sauder,D.N., Iijima,M., Wolff,K., Peharnberger,H., and Stingl,G. (1983). Mechanism of UV-B-induced impairment of the antigen-presenting capacity of murine epidermal cells. J Immunol 130, 1586-1591.

27. Tucci, A., James, H., Chicheportiche, R., Bonnefoy, J.Y., Dayer, J.M., and Zubler, R.H., 1992, J.Immunol. 148,2778-2784.

28. Ushio S, Namba M, Okura T, Hattori K, Nukada Y, Akita K, Tanabe F, Konishi K, Micallef M, Fujii M, Torigoe K, Tanimoto T, Fukuda S, Ikeda M, Okamura H, Kurimoto M. J Immunol 1996 Jun 1;156(11):4274-9

29. Yoshimoto T, Takeda, K, Tanaka, T, Ohkusu, K, Kashiwamura, S, Okamura, H, Akira, S and Nakanishi, K (1998), J. Immunol. 161, 3400-3407.

30. Xu et al. (1998). J. of Interferon and Cytokine Research 18:653-659.

**Claims**

1. Use of an IL-18 inhibitor for the manufacture of a medicament for treatment and/or prevention of a type IV hypersensitivity disorder, wherein the inhibitor of IL-18 is selected from
   IL-18-binding protein (IL-18BP),
   an IL-18BP fused to an immunoglobulin or a fragment thereof, wherein the fused protein binds to IL-18,
   a functional derivative of IL-18BP, wherein IL-18BP is linked to polyethylene glycol (PEG),
   or active fraction thereof having a similar activity as IL-18BP.

2. The use according to claim 1, wherein the hypersensitivity disorder is delayed type hypersensitivity.

3. The use according to claim 1 or 2, wherein the hypersensitivity disorder is contact hypersensitivity.

4. The use according to any of the preceding claims, wherein the medicament further comprises an interferon, for simultaneous, sequential or separate use.

5. The use according to claim 4, wherein the interferon is interferon-$\beta$.

6. The use according to any of the preceding claims, wherein the medicament further comprises an inhibitor of Tumor Necrosis Factor (TNF) for simultaneous, sequential or separate use.

7. The use according to claim 6, wherein the inhibitor of TNF is TBP I and/or TBP II.

8. The use according to any of the preceding claims, wherein the medicament further comprises an anti-inflammatory agent, for simultaneous, sequential or separate use.

9. The use according to claim 8, wherein the anti-inflammatory agent is COX-inhibitor.

10. The use according to any of the preceding claims, wherein the medicament further comprises an anti-allergic agent for simultaneous, sequential or separate use.

11. The use according to any of the preceding claims, wherein the inhibitor of IL-18 is used in an amount of 0.001 to 1000 mg/kg of body weight, or 0.01 to 100 mg/kg of body weight or 0.1 to 10 mg/kg of body weight or 5 mg/kg of body weight.

12. The use according to any of the preceding claims, wherein the IL-18 inhibitor is administered subcutaneously.

13. The use according to any of claims 1-11, wherein the IL-18 inhibitor is administered intramuscularly.

14. The use according to any of claims 1-11, wherein the IL-18 inhibitor is administered topically.

15. Use of an expression vector comprising the coding sequence of an inhibitor of IL-18 in the manufacture of a medicament for the treatment and/or prevention of a type IV hypersensitivity disorder, wherein the inhibitor of IL-18 is selected from IL-18-binding protein (IL-18BP), fused protein, functional derivative as defined in claim 1, or active fraction thereof having a similar activity as IL-18BP.

16. Use of a vector for inducing and/or enhancing the endogenous production of an inhibitor of IL-18 in a cell in the manufacture of a medicament for the treatment and/or prevention of a type IV hypersensitivity disorder, wherein the inhibitor of IL-18 is selected from IL-18-binding protein (IL-18BP), fused protein, functional derivative as defined in claim 1, or active fraction thereof having a similar activity as IL-18BP.

17. Use of a cell that has been genetically modified to produce an inhibitor of IL-18 in the manufacture of a medicament for the treatment and/or prevention of a type IV hypersensitivity disorder, wherein the inhibitor of IL-18 is selected from IL-18-binding protein, fused protein, functional derivative as defined in claim 1, or active fraction thereof having a similar activity as IL-18BP.

**Patentansprüche**

1. Verwendung eines IL-18 Inhibitors zur Herstellung eines Medikaments zur Behandlung und/oder Vorbeugung einer Typ IV Überempfindlichkeitsstörung, wobei der IL-18 Inhibitor ausgewählt ist aus dem IL-18 bindenden Protein (IL-18BP),
einem an Immunglobulin fusionierten oder einem Fragment davon IL-18BP, wobei das fusionierte Protein an IL-18 bindet,
einem funktionalen IL-18 Derivat, wobei IL-18BP an Polyethylenglykol (PEG) gebunden ist,
oder einer aktiven Fraktion davon, welche eine ähnliche Aktivität wie IL-18BP aufweist.

2. Verwendung nach Anspruch 1, wobei die Überempfindlichkeitsstörung eine verzögerte Überempflindlichkeit ist.

3. Verwendung nach Anspruch 1 oder 2, wobei die Überempfindlichkeitsstörung eine Kontakt-Überempfindlichkeit ist.

4. Verwendung nach einem der vorhergehenden Ansprüche, wobei das Medikament ferner ein Interferon zur gleichzeitigen, sequentiellen oder getrennten Anwendung umfasst.

5. Verwendung nach Anspruch 4, wobei das Interferon Interferon-$\beta$ ist.

6. Verwendung nach einem der vorhergehenden Ansprüche, wobei das Medikament ferner einen Tumor-Nekrose-Faktor (TNF) Inhibitor zur gleichzeitigen, sequentiellen oder getrennten Anwendung umfasst.

7. Verwendung nach Anspruch 6, wobei der TNF Inhibitor TBP I und/oder TBP II ist.

8. Verwendung nach einem der vorhergehenden Ansprüche, wobei das Medikament ferner einen entzündungshemmenden Wirkstoff zur gleichzeitigen, sequentiellen oder getrennten Anwendung umfasst.

9. Verwendung nach Anspruch 8, wobei der entzündungshemmende Wirkstoff ein COX-Inhibitor ist.

10. Verwendung nach einem der vorhergehenden Ansprüche, wobei das Medikament ferner ein Antiallergikum zur gleichzeitigen, sequentiellen oder getrennten Nutzung umfasst.

11. Verwendung nach einem der vorhergehenden Ansprüche, wobei der IL-18 Inhibitor in einer Menge von 0,001 bis 1000 mg/kg Körpergewicht, oder von 0,01 bis 100 mg/kg Körpergewicht oder von 0,1 bis 10 mg/kg Körpergewicht oder 5 mg/kg Körpergewicht verwendet wird.

12. Verwendung nach einem der vorhergehenden Ansprüche, wobei der IL-18 Inhibitor subkutan verabreicht wird.

13. Verwendung nach einem der Ansprüche 1-11, wobei der IL-18 Inhibitor intramuskulär verabreicht wird.

14. Verwendung nach einem der Ansprüche 1-11, wobei der IL-18 Inhibitor topisch verabreicht wird.

15. Verwendung eines Expressionsvektors umfassend die kodierende Sequenz eines IL-18 Inhibitors bei der Herstellung eines Medikaments zur Behandlung und/oder Vorbeugung einer Typ IV Überempfindlichkeitsstörung, wobei der IL-18 Inhibitor ausgewählt ist aus einem IL-18 bindenden Protein (IL-18BP), fusionierten Protein, funktionellen Derivat wie in Anspruch 1 definiert, oder einer aktiven Fraktion davon, welche eine ähnliche Aktivität wie IL-18BP aufweist.

16. Verwendung eines Vektors zum Induzieren und/oder Erhöhen der endogenen Herstellung eines IL-18 Inhibitors in einer Zelle bei der Herstellung eines Medikaments zur Behandlung und/oder Vorbeugung einer Typ IV Überempfindlichkeitsstörung, wobei der IL-18 Inhibitor ausgewählt ist aus einem IL-18 bindenden Protein (IL-18BP), fusionierten Protein, funktionellen Derivat wie in Anspruch 1 definiert, oder einer aktiven Fraktion davon, welche eine ähnliche Aktivität wie IL-18BP aufweist.

17. Verwendung einer Zelle, welche genetisch verändert wurde, um einen IL-18 Inhibitor herzustellen, bei der Herstellung eines Medikaments zur Behandlung und/oder Vorbeugung einer Typ IV Überempfindlichkeitsstörung, wobei der IL-18 Inhibitor ausgewählt ist aus einem IL-18 bindenden Protein (IL-18BP), fusionierten Protein, funktionellen Derivat wie in Anspruch 1 definiert, oder einer aktiven Fraktion davon, welche eine ähnliche Aktivität wie IL-18BP aufweist.

## Revendications

1. Utilisation d'un inhibiteur de IL-18 dans la fabrication d'un médicament destiné au traitement prophylactique et/ou thérapeutique d'un trouble d'hypersensibilité de type IV, l'inhibiteur de IL-18 étant choisi parmi
   la protéine de liaison de IL-18 (IL-18BP),
   une protéine IL-18BP fusionnée à une immunoglobuline ou l'un de ses fragments, où la protéine fusionnée se lie à IL-18,
   un dérivé fonctionnel de IL-18BP, où IL-18BP est liée au polyéthylène glycol (PEG),
   ou l'une de leurs fractions actives présentant une activité similaire à celle de IL-18BP.

2. Utilisation selon la revendication 1, où le trouble d'hypersensibilité est l'hypersensibilité de type retardé.

3. Utilisation selon la revendication 1 ou 2, où le trouble d'hypersensibilité est l'hypersensibilité de contact.

4. Utilisation selon l'une quelconque des revendications précédentes, où le médicament comprend en outre un interféron, pour utilisation simultanée, séquentielle ou séparée.

5. Utilisation selon la revendication 4, où l'interféron est l'interféron β.

6. Utilisation selon l'une quelconque des revendications précédentes, où le médicament comprend en outre un inhibiteur du Facteur de nécrose tumorale (TNF), pour utilisation simultanée, séquentielle ou séparée.

7. Utilisation selon la revendication 6, où l'inhibiteur de TNF est TBP I et/ou TBP II.

8. Utilisation selon l'une quelconque des revendications précédentes, où le médicament comprend en outre un agent anti-inflammatoire, pour utilisation simultanée, séquentielle ou séparée.

9. Utilisation selon la revendication 8, où l'agent anti-inflammatoire est un inhibiteur de COX.

**10.** Utilisation selon l'une quelconque des revendications précédentes, où le médicament comprend en outre un agent antiallergique, pour utilisation simultanée, séquentielle ou séparée.

**11.** Utilisation selon l'une quelconque des revendications précédentes, où l'inhibiteur de IL-18 est utilisé à une teneur comprise entre 0,001 et 1000 mg/kg de poids corporel, ou entre 0,01 et 100 mg/kg de poids corporel, ou entre 0,1 et 10 mg/kg de poids corporel, ou 5 mg/kg de poids corporel.

**12.** Utilisation selon l'une quelconque des revendications précédentes, où l'inhibiteur de IL-18 est administré par voie sous-cutanée.

**13.** Utilisation selon l'une quelconque des revendications 1 à 11, où l'inhibiteur de IL-18 est administré par voie intramusculaire.

**14.** Utilisation selon l'une quelconque des revendications 1 à 11, où l'inhibiteur de IL-18 est administré par voie topique.

**15.** Utilisation d'un vecteur d'expression comprenant la séquence codante d'un inhibiteur de IL-18 dans la fabrication d'un médicament destiné au traitement prophylactique et/ou thérapeutique d'un trouble d'hypersensibilité de type IV, où l'inhibiteur de IL-18 est choisi parmi la protéine de liaison de IL-18 (IL-18BP), une protéine fusionnée, un dérivé fonctionnel selon la revendication 1, ou l'une de leurs fractions actives présentant une activité similaire à celle de IL-18BP.

**16.** Utilisation d'un vecteur destiné à l'induction et/ou l'amplification de la production endogène d'un inhibiteur de IL-18 dans une cellule dans la fabrication d'un médicament destiné au traitement prophylactique et/ou thérapeutique d'un trouble d'hypersensibilité de type IV, où l'inhibiteur de IL-18 est choisi parmi la protéine de liaison de IL-18 (IL-18BP), une protéine fusionnée, un dérivé fonctionnel selon la revendication 1, ou l'une de leurs fractions actives présentant une activité similaire à celle de IL-18BP.

**17.** Utilisation d'une cellule génétiquement modifiée pour produire un inhibiteur de IL-18 dans la fabrication d'un médicament destiné au traitement prophylactique et/ou thérapeutique d'un trouble d'hypersensibilité de type IV, où l'inhibiteur de IL-18 est choisi parmi la protéine de liaison de IL-18 (IL-18BP), une protéine fusionnée, un dérivé fonctionnel selon la revendication 1, ou l'une de leurs fractions actives présentant une activité similaire à celle de IL-18BP.

Fig. 1

Fig. 2

Fig. 3

Fig. 4

* p < 0.05, ** p < 0.01

| Cell type | Marker | vehicle | IL-18BP | non-sens. |
|---|---|---|---|---|
| hematopoietic | CD45 | 4.45 / 100 % | 3.96 / 88.9 % | 2.73 / 61.3 % |
| αβ T | CD4 | 0.07 / 100 % | 0.05 / 71.4 % | 0.02 / 28.6 % |
| αβ T | CD8 | 0.24 / 100 % | 0.17 / 70.8 % | 0.02 / 8.3 % |
| NK | NK1.1 | 0.11 / 100 % | 0.07 / 59.4 % | 0 / 0 % |
| neutrophil | GR-1 | 0.54 / 100 % | 0.36 / 66.7 % | 0.02 / 3.7 % |
| monocyte / macrophage | CD11b hi / CD11c neg | 0.53 / 100 % | 0.42 / 79.2 % | 0.03 / 5.7 % |

green: % of total cells    black: relative proportion

Fig. 5

Fig. 6

| IFNγ + | vehicle | IL-18BP | non-sens. | |
|--------|---------|---------|-----------|---|
| all | 100 % | 77.8 % | - | relative |
| | 0.18 | 0.14 | n.d. | |
| CD4 | 0.05 | 0.03 | n.d. | % of total |
| CD8 | 0.13 | 0.11 | n.d. | |
| NK | n.d. | n.d. | n.d. | |
| γδ T | n.d. | n.d. | n.d. | |
| PMA / Ionomycin stimulation for 4h | | | | |

Fig. 7

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9909063 A
- EP 110969 A
- EP 0974600 A
- EP 0864585 A
- US 4959314 A
- US 4588585 A
- US 4737462 A, Mark
- US 5116943 A, Koths
- US 4965195 A, Namen
- US 4879111 A, Chong
- US 5017691 A, Lee
- US 4904584 A, Shaw
- WO 0076310 A

- WO 9953049 A
- US 6162963 A
- AU 5336100
- WO 9213095 A
- WO 0103737 A
- WO 9955377 A
- EP 914431 A
- EP 308378 A
- EP 398327 A
- EP 433900 A
- WO 0100229 A
- WO 9109955 A

### Non-patent literature cited in the description

- **AUSUBEL et al.** Current Protocols in Molecular Biology. Interscience, 1987
- **ALTSCHUL S F et al.** *J Mol Biol,* 1990, vol. 215, 403-410
- **ALTSCHUL S F et al.** *Nucleic Acids Res.,* 1997, vol. 25, 389-3402
- **ANTONOPOULOS, C. ; M. CUMBERBATCH ; R. J. DEARMAN ; R. J. DANIEL ; I. KIMBER ; R. W. GROVES.** Functional caspase-1 is required for Langerhans cell migration and optimal contact sensitization in mice. *J Immunol,* 2001, vol. 166, 3672-3677
- **BOUR, H. et al.** Major histocompatibility complex class I-restricted CD8 + T cells and class II-restricted CD4 + T cells, respectively, mediate and regulate contact sensitivity to dinitrofluorobenzene. *Eur. J. Immunol.,* 1995, vol. 25, 3006-3010
- **CHATER, K. F. et al.** *Sixth International Symposium on Actinomycetales Biology,* 1986, 45-54
- **CONTI, B. ; J. W. JAHNG ; C. TINTI ; J. H. SON ; T. H. JOH.** Induction of interferon-gamma inducing factor in the adrenal cortex. *J. Biol. Chem.,* 1997, vol. 272, 2035-2037
- **CUMBERBATCH, M. ; R. J. DEARMAN ; C. ANTONOPOULOS ; R. W. GROVES ; I. KIMBER.** Interleukin (IL)-18 induces Langerhans cell migration by a tumour necrosis factor-alpha- and IL-1beta-dependent mechanism. *Immunology,* 2001, vol. 102, 323-330
- **DEVEREUX J et al.** *Nucleic Acids Res,* 1984, vol. 12, 387-395

- **DIDONATO, J A ; HAYAKAWA, M ; ROTHWARF, D M ; ZANDI, E ; KARIN, M.** *Nature,* 1997, vol. 388, 16514-16517
- **ELLIOTT, M.J. ; MAINI, R.N. ; FELDMANN, M. ; LONG-FOX, A. ; CHARLES, P. ; BIJL, H. ; WOODY, J.N.** *Lancet,* 1994, vol. 344, 1125-1127
- **ENGELMANN, H. ; D. NOVICK ; D. WALLACH.** Two tumor necrosis factor-binding proteins purified from human urine. Evidence for immunological cross-reactivity with cell surface tumor necrosis factor receptors. *J. Biol. Chem.,* 1990, vol. 265, 1531-1536
- **GARRIGUE JL ; NICOLAS JF ; FRAGINALS R ; BENEZRA C ; BOUR H ; SCHMITT D.** *Contact Dermatitis,* April 1994, vol. 30 (4), 231-7
- **GRABBE, S. ; SCHWARZ, T.** Immunoregulatory mechanisms involved in elicitation of allergic contact hypersensitivity. *Immunol. Today,* 1998, vol. 19, 37-44
- **HABU et al.** *J. Immunol.,* 2001, vol. 166, 5439-5347
- **HANIFIN et al.** *J. Am. Acad. Dermatol.,* 1993, vol. 28, 189
- **KIM SH ; EISENSTEIN M ; REZNIKOV L ; FANTUZZI G ; NOVICK D ; RUBINSTEIN M ; DINARELLO CA.** Structural requirements of six naturally occurring isoforms of the IL-18 binding protein to inhibit IL-18. *Proc Natl Acad Sci U S A,* 2000, vol. 97, 1190-1195
- **KIMBER, I. ; M. CUMBERBATCH.** Stimulation of Langerhans cell migration by tumor necrosis factor alpha (TNF-alpha). *J Invest Dermatol.,* 1992, vol. 99, 48S-50S

- **MALISZEWSKI, C. R. ; T. A. SATO ; T. VANDEN BOS ; S. WAUGH ; S. K. DOWER ; J. SLACK ; M. P. BECKMANN ; K. H. GRABSTEIN.** Cytokine receptors and B cell functions. I. Recombinant soluble receptors specifically inhibit IL-1- and IL-4-induced B cell activities in vitro. *J. Immunol.,* 1990, vol. 144, 3028-3033
- **MICALLEF, M. J. ; T. OHTSUKI ; K. KOHNO ; F. TANABE ; S. USHIO ; M. NAMBA ; T. TANIMOTO ; K. TORIGOE ; M. FUJII ; M. IKEDA.** Interferon-gamma-inducing factor enhances T helper 1 cytokine production by stimulated human T cells: synergism with interleukin-12 for interferon-gamma production. *Eur-J-Immunol,* 1996, vol. 26, ISSN 0014-2980, 1647-51
- **NAKAMURA K ; OKAMURA H ; WADA M ; NAGATA K ; TAMURA T.** *Infect Immun,* February 1989, vol. 57 (2), 590-5
- **NOVICK, D ; KIM, S-H ; FANTUZZI, G ; REZNIKOV, L ; DINARELLO, C ; RUBINSTEIN, M.** *Immunity,* 1999, vol. 10, 127-136
- **OKAMURA H ; NAGATA K ; KOMATSU T ; TANIMOTO T ; NUKATA Y ; TANABE F ; AKITA K ; TORIGOE K ; OKURA T ; FUKUDA S et al.** *Infect Immun,* October 1995, vol. 63 (10), 3966-72
- **PARNET, P ; GARKA, K E ; BONNERT, T P ; DOWER, S K ; SIMS, J E.** *J. Biol. Chem.,* 1996, vol. 271, 3967-3970
- **REINHOLD et al.** *Lancet,* 1990, vol. 335, 1282
- **ROTHE H ; JENKINS NA ; COPELAND NG ; KOLB H.** *J Clin Invest,* 01 February 1997, vol. 99 (3), 469-74
- **SCHULER,G. ; STEINMAN,R.M.** Murine epidermal Langerhans cells mature into potent immunostimulatory dendritic cells in vitro. *J Exp. Med.,* 1985, vol. 161, 526-546
- **STINGL,L.A. ; SAUDER,D.N. ; LIJIMA,M. ; WOLFF,K. ; PEHARNBERGER,H. ; STINGL,G.** Mechanism of UV-B-induced impairment of the antigen-presenting capacity of murine epidermal cells. *J Immunol,* 1983, vol. 130, 1586-1591
- **TUCCI, A. ; JAMES, H. ; CHICHEPORTICHE, R. ; BONNEFOY, J.Y. ; DAYER, J.M. ; ZUBLER, R.H.** *J.Immunol.,* 1992, vol. 148, 2778-2784
- **USHIO S ; NAMBA M ; OKURA T ; HATTORI K ; NUKADA Y ; AKITA K ; TANABE F ; KONISHI K ; MICALLEF M ; FUJII M.** *J Immunol,* 01 June 1996, vol. 156 (11), 4274-9
- **YOSHIMOTO T ; TAKEDA, K ; TANAKA, T ; OHKUSU, K ; KASHIWAMURA, S ; OKAMURA, H ; AKIRA, S ; NAKANISHI, K.** *J. Immunol.,* 1998, vol. 161, 3400-3407
- **XU et al.** *J. of Interferon and Cytokine Research,* 1998, vol. 18, 653-659